# EUROPEAN PATENT APPLICATION

(11) **EP 4 123 307 A1**
(43) Date of publication of application: **25.01.2023**
(21) Application number: 21187208.0
(22) Date of filing: 22.07.2021
(51) Int. Cl.: G01N 33/68, A61K 9/127

(54) **METHODS FOR STRUCTURE DETERMINATION AND DRUG DESIGN USING SALIPRO PARTICLES**

(71) Applicant: Salipro Biotech AB, 114 28 Stockholm (SE)
(72) Inventor: Löving, Robin, 112 19 Stockholm (SE); Frauenfeld, Jens, 11418 Stockholm (SE)
(74) Representative: Cohausz & Florack

(57) **Abstract**

The invention relates to a method for determining a 2D or 3D structure of a hydrophobic agent such as a membrane protein by using saposin lipoprotein particles comprising apart from the particle scaffold components and the hydrophobic agent at least a first binding agent. The invention further concerns a method for identifying a new drug candidate that binds to a hydrophobic agent and/or lipids which also makes use of saposin lipoprotein particles comprising particle scaffold components, a hydrophobic agent and a first binding agent. The saposin lipoprotein particles, optionally for use in the methods of the invention, comprise a first binding agent specifically binding to the particle scaffold, i.e. the saposin-like protein or a derivative form thereof, and/or lipids. Lastly, the invention concerns uses of the saposin lipoprotein particles of the invention, and uses of the first binding agent in connection with the saposin lipoprotein particles.

## Description

### TECHNICAL FIELD OF THE INVENTION

The invention relates to methods for determining a 2D or preferably a 3D structures of hydrophobic agents, in particular membrane proteins and interaction partners, by using saposin lipoprotein particles, also referred to as Salipro particles herein, that are complexed with binding agents such as antibodies that specifically bind to the saposin or lipid components of the Salipro particles. The invention further provides methods for studying or identifying a biologically active agent that binds to a hydrophobic agent and/or lipids which also makes use of saposin lipoprotein particles and is particularly useful in drug design applications. The invention also concerns certain uses of the structures obtained by the structure determination methods of the invention and uses of the saposin lipoprotein particles complexed with the binding agent.

### BACKGROUND OF THE INVENTION

Membranes surround all living cells, many organelles (e.g., mitochondria) and form the envelopes of many viruses (e.g., HIV). Biological membranes serve as semi-permeable barriers to preserve the contents they enclose. The membrane constituents, in particular membrane proteins, determine and regulate interactions with the environment. These include biological processes such as signal transduction, transport of molecules, energy metabolism, formation of cell-cell contacts and cellular homeostasis. These interactions typically involve membrane proteins such as receptors and transporters. Membrane proteins of viral envelopes also serve to mediate interactions with the environment such as the host's cell membranes allowing the viral capsid and the viral genome to enter and infect the host cells.

Membrane proteins are encoded by approximately 30% of all ORFs in eubacterial, archaean, and eukaryotic organisms (Wallin and von Heijne, Protein Science 1998 Apr; 7 (4):1029-38) and thus represent roughly one quarter of a eukaryotic cell's protein repertoire.

Membrane proteins are of great interest for the research in the life sciences and also for drug discovery programs *inter alia* because of their functions in regulating the cell's interactions with the environment. In fact, the majority of drugs, i.e. more than 60%, target membrane proteins (Overington et al., Nature Reviews Drug Discovery 5, 993-996, 2006). Membrane proteins are, however, challenging to study in their native forms due to their inherent instability, insolubility and tendency to aggregate when extracted from their natural lipid bilayer environment.

For research purposes and in particular drug development programs, it is therefore crucial that on the one hand hydrophobic agents including membrane proteins can be subjected to structural biology methods such as X-ray crystallography, high-resolution cryo electron microscopy (Cryo-EM), nuclear magnetic resonance spectroscopy (NMR), negative-stain electron microcopy, and small-angle x-scattering (SAXS), without impairing the native structure of the hydrophobic agent that depends on critical interactions with its natural lipid bilayer environment. The 3D structures obtained by these methods enable structure-based drug design using the hydrophobic agent as a target molecule. On the other hand, it is also crucial that hydrophobic agents can be subjected to state-of-the-art technology platforms and assay systems without altering and/or impairing the native function of the hydrophobic agent so that various activity data can be acquired. State-of-the-art technology platforms and assay systems include, e.g., surface plasmon resonance (SPR), phage display, B-cell sorting, fluorescent activated cell sorting (FACS), bio-layer interferometry (BLI), radioligand-binding, SDS-PAGE, Western Blotting, multiple-angle light scattering (MALS), quasielastic light scattering (QELS), size-exclusion chromatography (SEC), mass spectrometry, analytical ultracentrifugation (AUC), high-throughput methods, high-throughput screenings, fragment-based libraries and drug design, DNA-encoded libraries, phage/yeast/ribosome/mRNA display technologies or any other display library technology, methods involving immunization, sorting, enrichment or selection of B-cells or hybridoma cells, any method to sort, select, or enrich tissue, cells, viruses, or bacteria, proteolysis targeting chimera (PROTAC) development, chimeric antigen receptors T-cell (CAR-T) development and enzyme-linked immunosorbend assays (ELISAs).

In the past, different carrier systems have been developed to stabilize hydrophobic agents, in particular membrane proteins in a lipid environment. Saposin lipoprotein particles are particularly suitable carrier systems for hydrophobic agents because they mimic the membrane environment found in biological membranes and stabilize membrane proteins in their native forms.

In this regard, WO 2014/095576 A1 showed for the first time that it is possible to incorporate purified, detergent-solubilized hydrophobic cargo molecules or purified, detergent-solubilized membrane proteins into saposin lipoprotein particles using detergent-solubilized and purified lipids together with saposin-like proteins or derivative forms thereof. Thus, WO 2014/095576 A1 describes saposin lipoprotein particles comprising a lipid binding polypeptide, lipids and a hydrophobic agent, wherein the hydrophobic agent is different from the lipids, and wherein the lipid binding polypeptide is a saposin-like protein or a derivative or truncated form thereof.

WO 2015/036549 A1 further describes saposin lipoprotein particles comprising certain antigens as hydrophobic agents. Specifically, WO 2015/036549 A1 teaches saposin lipoprotein particles comprising at least one hydrophobic or partially hydrophobic antigen molecule from a virus, a bacterium, fungus, protozoan, parasite, a human neoplastic cell or an animal neoplastic, tumour or cancer cell, reconstituted in a lipid membrane mimicking environment formed by a lipid binding polypeptide such that the antigen molecules(s) is/are assembled therein.

WO 2018/033657 A1 describes for the first time a process for preparing a library of saposin lipoprotein particles, wherein the particles are generated from crude membranes, i.e. native membrane material. The library of the saposin lipoprotein particles differ in their lipid and/or protein composition.

WO 2020/212423 A1 teaches to perform the assembly of saposin lipoprotein particles on a support, in particular by having the hydrophobic agent or the saposin-like protein comprise a binding moiety, which can be an affinity tag such as His-tag or strepatavidin via which it can be immobilized (see WO 2020/212423 A1, claims 1 and 2 and p. 38,1. 4 to p. 40,1. 5).

Although the prior art saposin lipoprotein particles are already useful for structural biology methods and biochemical assays to analyze the hydrophobic agents contained therein, there always is a need for further improvement. Biochemical assays, for example, often require that the assay analyte can be detected specifically at low levels and sometimes even in a spatio-temporal manner. Structural biology methods including X-ray crystallography and cryo-EM are demanding in terms of time and cost. It is also often very difficult to impossible to obtain high-resolution structures or to determine the structure of flexible and/or unstable proteins, small proteins as wells as large proteins or protein complexes, in particular in a membrane-bound native state. A further difficulty in determining the structure of hydrophobic agents such as membrane proteins is their inherent insolubility in aqueous buffer systems.

### SUMMARY OF THE INVENTION

In light of the above-described background, it is an object of the present invention to provide an efficient method allowing to better determine a full or partial 2D or 3D structure, preferably a full or partial 3D structure, of a hydrophobic agent. Specifically, it is an object of the present invention to provide a method which is particularly suitable to determine a full or partial 2D or 3D structure, preferably a full or partial 3D structure, of a hydrophobic agent by X-ray crystallography, cryo-EM and/or NMR. It is also an object of the present invention to provide an efficient method for studying and identifying new biologically active agents, in particular new drug candidates against a hydrophobic agent.

A further object of the invention is to provide carriers which are particularly suitable for investigating, making accessible or delivering hydrophobic agents contained therein. In particular, it is an object of the present invention to provide carriers for hydrophobic agents which are particularly suitable for use in structural biology methods, analytical assays and/or medical treatments.

These objects are achieved by the methods of claims 1 and 6, the saposin lipoprotein particles according to claim 7, and the uses according to claims 14 and 15.

The invention provides a method comprising determining a full or partial 2D or 3D structure, preferably a full or partial 3D structure, of
- a hydrophobic agent, and/or
- a second agent interacting with the hydrophobic agent, and/or
- a binding site between
- a biologically active agent and a hydrophobic agent, or
- a biologically active agent and lipids, or
- a biologically active agent a hydrophobic agent and lipids;
the method comprising the following steps:
a) providing a particle comprising the following components
   - a saposin-like protein or a derivative form thereof;
   - lipids;
   - a hydrophobic agent wherein the hydrophobic agent is different from the lipids; and
   - a first binding agent that is bound to
      - the saposin-like protein or the derivative form thereof; and/or
      - the lipids;
   - optionally a second binding agent wherein the second binding agent is bound to the hydrophobic agent and optionally lipids; and
   - optionally a biologically active agent interacting with the hydrophobic agent and/or the lipids;
b) determining the full or partial 2D or 3D structure of the particle of step a) using a structural biology method for structure elucidation.

Preferably, the method is for determining such a full or partial 2D or 3D structure. The employed saposin lipoprotein particles make the method of the invention particularly suitable for this purpose. While the essential particle forming components, i.e. the saposin-like protein or the derivative form thereof and the lipids, stabilize the hydrophobic agent in an environment mimicking its natural surroundings, it has been surprisingly found that the first binding agent and optionally the second binding agent (the second agent specified in the claims as interacting with the hydrophobic agent and optionally lipids) not only increase the particle size, but thereby also enhance the rigidity of the particle, influence the orientation of the particle when present on a support, and/or alter (increase or decrease) the symmetry of the particle. In doing so, structure determination of the hydrophobic agent, optionally including interaction partners, is surprisingly facilitated by the invention, in particular when using as structural biology methods in step b) X-ray crystallography, cryo-EM or NMR.

Using first binding agents which bind to the saposin-like protein or the derivative form thereof and/or the particle lipids has the additional advantage that they allow to influence the particle characteristics in a manner independent of the hydrophobic agent contained therein.

The use of Salipro particles complexed with a first binding agent and a second binding agent is particularly advantageous for determining a full or partial 2D or 3D structure of a hydrophobic agent, optionally including interaction partners. By using appropriate second binding agents also the hydrophobic agent contained in the saposin lipoprotein particle can be stabilized in its structural conformation and in particular flexible conformations can be held in place. At the same time, the second binding agent further increases the particle size and has an impact on the orientation of the particles when present on a support, and/or the alter (increase or decrease) of the particles which surprisingly further supports structure determination, in particular when using as structural biology methods in step b) X-ray crystallography, cryo-EM or NMR.

The invention further provides a method for studying or identifying a biologically active agent, in particular a new drug candidate, that binds to a hydrophobic agent, the method comprising:
a. obtaining one or more partial or full 2D or 3D structures, preferably one or more partial or full 3D structures of a particle comprising
   - a saposin-like protein or a derivative form thereof;
   - lipids;
   - a hydrophobic agent wherein the hydrophobic agent is different from the lipids;
   - a first binding agent wherein the binding agent is bound to
      - the saposin-like protein or the derivative form thereof; and/or
      - the lipids;
   - optionally a second binding agent wherein the second binding agent is bound to the hydrophobic agent and optionally the lipids; and
   - optionally a drug molecule interacting with the hydrophobic agent and/or the lipids;
b. modeling the binding of at least one biologically active agent to the hydrophobic agent using the one or more 2D or 3D structures obtained in step a.;
c. selecting one or more biologically active agents which are modeled to selectively bind to the hydrophobic agent in step b.; and
d. optionally, confirming the selective binding of the one or more biologically active agent selected in step c. to the hydrophobic agent in the particle of step a. by contacting the particle with the selected one or more biologically active agents and measuring the binding between the hydrophobic agent in the particle and the selected one or more drug candidates and/or the biological activity of the hydrophobic agent.

The biologically active agent can be any agent that shows biological activity such as a small molecule compound or a larger biological molecule such as a protein or a (poly)nucleotide agent. In particular, the biologically active agent can be a pharmaceutically active agent, in particular a drug candidate, or an active agent useful in agronomical or environmental applications.

The method of the invention is particularly suitable for identifying new drug candidates that bind to hydrophobic agent drug targets. This is largely due to the structure-based drug design step b. based on the partial or full 2D or 3D structures obtained with the special saposin lipoprotein particles of the invention. Step a. of this method of the invention includes the more generally described method of the invention. Thus, step a. confers the same advantageous effects as described above in connection with the more general method of the invention.

The saposin lipoprotein particles of the invention comprise
- a saposin-like protein or a derivative form thereof;
- lipids;
- a hydrophobic agent wherein the hydrophobic agent is different from the lipids;
- a first binding agent wherein the first binding agent is bound to
   - the saposin-like protein or the derivative form thereof and/or
   - the lipids;
- optionally
   - a second binding agent wherein the second binding agent is bound to the hydrophobic agent; and/or
   - a drug molecule interacting with the hydrophobic agent.

The particles of the invention are particularly suitable for membrane protein research, in particular structural biology methods as described herein, but also for proteomics, medical applications such as pharmaceutical compositions and vaccine formulations, therapeutic treatments, prophylactic treatments, diagnostic treatments and cosmetic treatments, *in vitro* methods for studying the hydrophobic agent of the particles of the invention. The reason for their broad application spectrum is that the first binding agent that is present in the particles provides a versatile "anchor" or "platform" to alter and adapt the particle properties.

As explained above, the additional presence of the first binding agent inherently increases the particle size and also has an impact on the rigidity of the particle, the orientation of the particle when present on a support, and/or the symmetry of the particle, which supports structure determination of the particle components, in particular when X-ray crystallography, cryo-EM or NMR, most preferably X-ray crystallography or cryo-EM, are used as structural biology methods.

The first binding agent can contain further functional moieties which allow to further adapt or modify the particle properties. For example, the first binding agent can facilitate multimerization of the particles by the presence of a dimerization or multimerization moiety, detection of the particles by the presence of a labeling moiety such as a fluorescent tag, and/or the capturing of further cargo molecules by the presence of a capture moiety, wherein the cargo molecule can be a drug molecule.

Since a particle of the invention typically contains multiple saposin-like proteins or derivative forms thereof as scaffold proteins and multiple lipid molecules, also multiple first binding agents will usually be present per particle. Thus, the particle properties can be altered quite considerably by the first binding agent. According to one embodiment, the particle comprises more than one, i.e. a plurality of first binding agents. The first binding agents can be the same or different from another.

Particles which contain a first binding agent and a second binding agent, i.e. two different binding agents, are particularly useful since these particles provide two independent versatile "anchors" or "platforms" to alter and adapt the particle properties.

The invention further concerns the use of a 2D or 3D structure, preferably the use of a 3D structure, of a hydrophobic agent determined according to the method of the invention for designing a drug molecule binding to the hydrophobic agent or to the hydrophobic agent and lipids.

A further use of the invention is the use of a first binding agent capable of binding to saposin-like protein or a derivative form thereof, and/or to lipids in a particle according to the invention for increasing the particle size, influencing the orientation of the particle on a solid support, and/or for influencing the rigidity of the particle to determine the full or partial 2D or 3D structure, preferably the full or partial 3D structure, of a hydrophobic agent in the particle.

Such a use of a first binding agent facilitates structure determination of a hydrophobic agent in the particle, in particular when using X-ray crystallography, cryo-EM or NMR, most preferably X-ray crystallography or cryo-EM, as structural biology method.

Another use of a first binding agent capable of binding to the saposin-like protein or a derivative form thereof, and/or lipids in the particle according to the invention concerns its use for multimerizing the particles, labeling the particles and/or capturing an additional cargo molecule.

Lastly, the invention concerns the use of a particle according to the invention in a pharmaceutical composition, in particular a vaccine formulation, in a therapeutic or prophylactic method, in particular in a method to prevent, treat or lessen the severity of a disease, in a diagnostic method, or a cosmetic treatment.

### DETAILED DESCRIPTION OF THE INVENTION

### Method to determine a 2D or 3D structure

This method aspect of the invention is suitable for determining a full or partial 2D or 3D structure, preferably a full or partial 3D structure of
- a hydrophobic agent, and/or
- a second binding agent interacting with the hydrophobic agent, and/or
- a binding site between
   - a biologically active agent and a hydrophobic agent, or
   - a biologically active agent and lipids, or
   - a biologically active agent a hydrophobic agent and lipids.

While a "3D structure" as used herein refers to a molecular structure, which is defined in x-, y- and z-dimensions, the term "2D structure" as used herein refers to a molecular structure which is only defined in two dimensions. As well known to the skilled person, the resolution of 2D or 3D structures vary considerably depending on the sample properties and the structure elucidation method employed.

A "full 2D or 3D structure" as used herein refers to a complete 2D or 3D structure of the target or study object, in particular the hydrophobic agent, optionally with interaction partners. A "partial 2D or 3D structure" means that only a part of the structure of the target or study object is solved. For example, only a structure of a part of the hydrophobic agent is solved in form of a 2D or 3D structure.

### Step a)

In step a) of the method of the invention a particle comprising the following components is provided
- a saposin-like protein or a derivative form thereof;
- lipids;
- a hydrophobic agent wherein the hydrophobic agent is different from the lipids;
- a first binding agent that is bound to
   - the saposin-like protein or the derivative form thereof; and/or
   - the lipids;
- optionally a second binding agent that is bound to the hydrophobic agent; and
- optionally a biologically active agent (in particular a drug molecule) interacting with the hydrophobic agent and/or the lipids;

Particles of this composition are particularly suitable for the structural biology methods in step b) of this method of the invention.

The singular forms "a", "an" and "the" as used herein include the plural forms unless the context clearly dictates otherwise. "A", "an" and "the" can thus be understood to mean "one or more" or "at least one". Thus, the saposin lipoprotein particle used in step a) of the method of the invention can comprise multiple and optionally even different saposin-like proteins or derivatives thereof, multiple and optionally even different hydrophobic agents, multiple and optionally even different first binding agents, multiple and optionally even different second binding agents, and multiple and optionally even different drug molecules. "Different" as used in this context means that the referred to molecules are chemically different, in particular different in terms of amino acid composition or sequence (in case of protein) or chemical structure (in case of lipid).

### A saposin-like protein or a derivative form thereof

The employed particle comprises one or more saposin-like proteins, also referred to as SAPLIPs herein, or derivative forms thereof. The term "saposin-like protein" (SAPLIP) is a technical term known to the skilled person and includes all members of the conserved saposin-like protein (SAPLIP) family of lipid interacting proteins. The SAPLIP family is characterized by the saposin-fold. This is a conserved alpha-helical three-dimensional structure that is stabilized by highly conserved intramolecular disulfide bonds (Munford et al. (1995), Journal of Lipid Research, vol. 36, no. 8, 1653-1663 and Bruhn (2005), Biochem J 389 (15): 249-257). Example members of the SAPLIP family according to the invention are described in Munford et al. (1995), Journal of Lipid Research, vol. 36, no. 8, 1653-1663 and Bruhn (2005), Biochem J 389 (15): 249-257, both of which are hereby incorporated by reference in their entirety.

In the ligand-free (i.e. detergent-free/lipid-free) "closed" state, the SAPLIPs adopt a monomeric, compact, four-helix bundle-type structure, the saposin fold. This fold is exemplified by the structure of the closed apo form of human saposin A (Protein Data Bank (PDB) ID code: 2DOB, Ahn et al. (2006) Protein Sci. 15: 1849-1857) or the structures of saposin C (PDB ID code: 1M12; de Alba et al. (2003) Biochemistry 42, 14729-14740), NK-lysin (PDB ID code: 1NKL; Liepinsh etal. (1997) Nat. Struct. Biol. 4, 793-795), amoebapore A (PDB ID code: 10F9) and granulysin (PDB ID code: 1L9L; Anderson et al. (2003) J. Mol. Biol. 325, 355-365), which are all nearly identical and easily super-imposable.

SAPLIPs undergo a conformational change upon binding to ligands such as lipids or detergent molecules. In the ligand-bound "open" conformation, SAPLIPs adopt a V-shaped or boomerang-shaped conformation with exposed hydrophobic surfaces that contact the bound lipids. The open conformation is exemplified by the saposin A detergent disc structure of the prior art (PDB ID code: 4DDJ; Popovic et al., PNAS, Vol. 109, No.8 (2012) 2908-2912) and the structure of saposin C bound to SDS detergent micelles (PDB ID code: 1SN6; Hawkins etal. (2005) J.Mol.Biol. 346: 1381-1392).

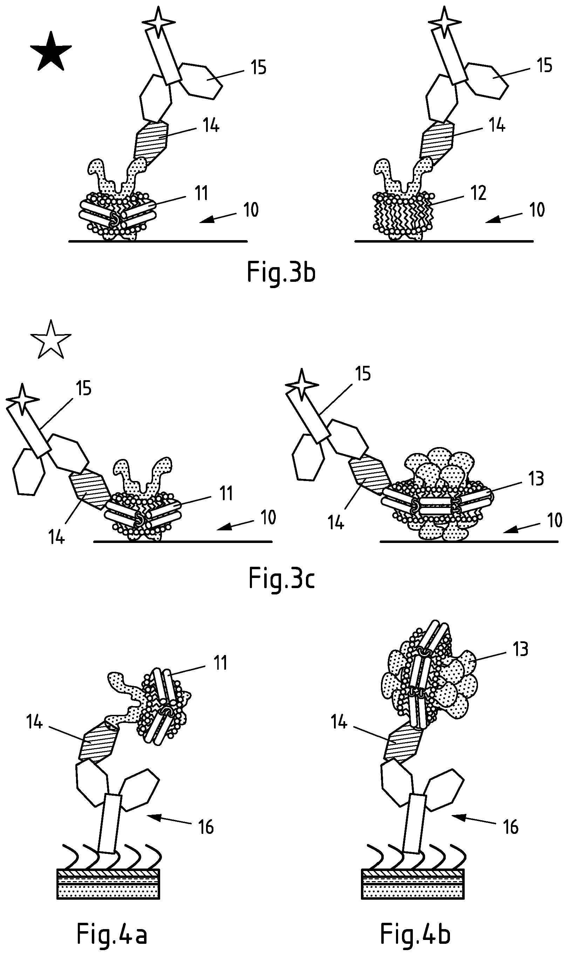

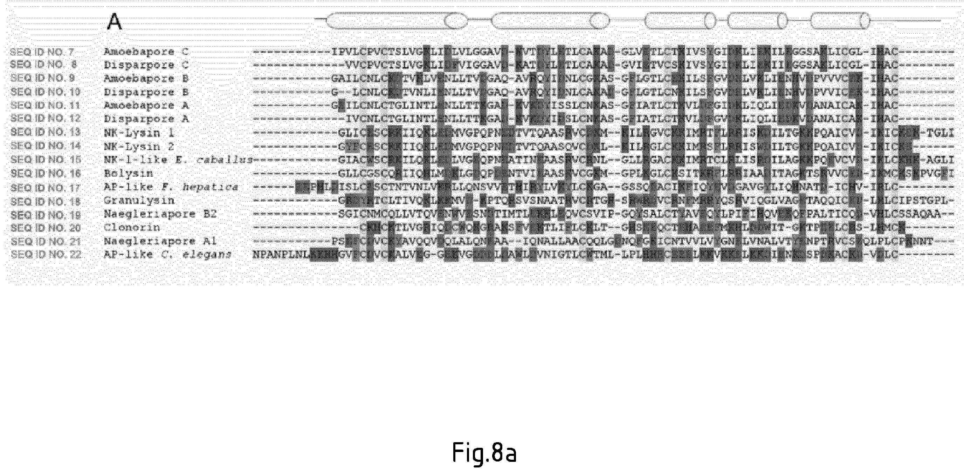

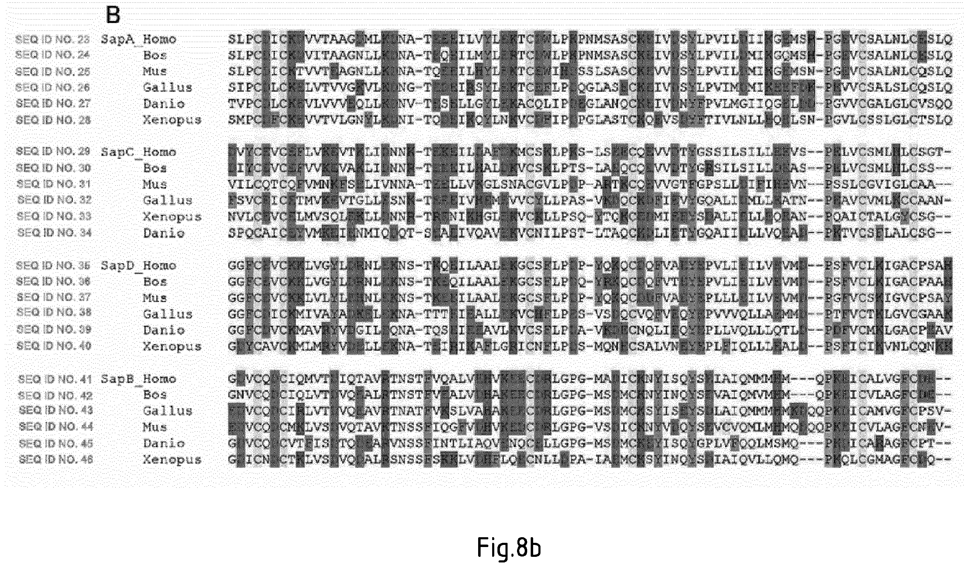

Whereas the ability to interact with lipids, the above-described amphipathic nature and three-dimensional structure is highly conserved among SAPLIPs, they are highly diverse on the amino acid sequence level, with sequence identities below the usual threshold of 25-30% identity to define homology (see the sequence comparison in Fig. 4A and 4B of Bruhn (2005), Biochem J 389 (15): 249-257, reproduced as Figure 8a, 8b herein).

Examples of SAPLIPs according to the invention are saposins A, B, C or D (for example from Homo sapiens [cf. SEQ ID NO. 1 to 4], Equus caballus, Bos taurus, Mus musculus, Oryctolagus cuniculus, Rattus norvegicus or Xenopus laevis); Surfactant protein B (for example from Homo sapiens, Canis familiaris, Mus musculus, Oryctolagus cuniculus, Ovis aries or Rattus norvegicus); Granulysin (for example from Homo sapiens; cf. SEQ ID NO. 5); NK-lysin (for example from Sus scrofa; cf. SEQ ID NO. 6); NK-lysin orthologues (for example from Equus caballus or Bos taurus); Amoebapores (for example from Entamoeba histolytica); Amoebapore orthologues (for example from Entamoeba dispar or Entamoeba invadens); Amoebapore-like protein (for example from Fasciola hepatica); Naegleriapores (for example from Naegleria fowleri); Clornorin (for example from Clonorchis sinensis); Prosaposin (for example from Homo sapiens, Equus caballus, Bos taurus, Mus musculus, Oryctolagus cuniculus, Rattus norvegicus or Xenopus laevis) and MSAP (for example from Homo sapiens).

The sequences of specific SAPLIPs used according to the invention are given in Fig. 4A and 4B of Bruhn (2005), Biochem J 389 (15): 249-257, which sequences are hereby specifically incorporated by reference and reproduced as Figure 8a, 8b herein. The sequences of particular SAPLIPs used according to the invention are given in the sequence listing as follows:

**Table 1:**

| **SEQ ID NO.** | **Source** | **Protein** | **Species** | **number of aa** |
|---|---|---|---|---|
| **1** | Public Domain | Saposin A | Homo sapiens | 81 |
| **2** | Public Domain | Saposin B | Homo sapiens | 79 |
| **3** | Public Domain | Saposin C | Homo sapiens | 80 |
| **4** | Public Domain | Saposin D | Homo sapiens | 78 |
| **5** | Public Domain | Granulysin | Homo sapiens | 145 |
| **6** | Public Domain | NK-lysin | Sus scrofa | 129 |
| **7** | Bruhn, 2005, Fig. 4A | Amoebapore C | Entamoeba histolytica | 77 |
| **8** | Bruhn, 2005, Fig. 4A | Disparpore C | Entamoeba dispar | 75 |
| **9** | Bruhn, 2005, Fig. 4A | Amoebapore B | Entamoeba histolytica | 77 |
| **10** | Bruhn, 2005, Fig. 4A | Disparpore B | Entamoeba dispar | 75 |
| **11** | Bruhn, 2005, Fig. 4A | Amoebapore A | Entamoeba histolytica | 77 |
| **12** | Bruhn, 2005, Fig. 4A | Disparpore A | Entamoeba dispar | 75 |
| **13** | Bruhn, 2005, Fig. 4A | NK-Lysin 1 | Sus scrofa | 83 |
| **14** | Bruhn, 2005, Fig. 4A | NK-Lysin 2 | Sus scrofa | 78 |
| **15** | Bruhn, 2005, Fig. 4A | NK-1-like *E*. *caballus* | Equus caballus | 83 |
| **16** | Bruhn, 2005, Fig. 4A | Bolysin | Bos taurus | 84 |
| **17** | Bruhn, 2005, Fig. 4A | AP-like *F. hepatica* | Fasciola hepatica | 81 |
| **18** | Bruhn, 2005, Fig. 4A | Granulysin | Homo sapiens | 83 |
| **19** | Bruhn, 2005, Fig. 4A | Naegleriapore B2 | Naegleria fowleri | 83 |
| **20** | Bruhn, 2005, Fig. 4A | Clonorin | Clonorchis sinensis | 73 |
| **21** | Bruhn, 2005, Fig. 4A | Naegleriapore A1 | Naegleria fowleri | 83 |
| **22** | Bruhn, 2005, Fig. 4A | AP-like C. *elegans* | Caenorhabditis elegans | 88 |
| **23** | Bruhn, 2005, Fig. 4B | Saposin A | Homo sapiens | 83 |
| **24** | Bruhn, 2005, Fig. 4B | Saposin A | Bos taurus | 83 |
| **25** | Bruhn, 2005, Fig. 4B | Saposin A | Mus musculus | 83 |
| **26** | Bruhn, 2005, Fig. 4B | Saposin A | Gallus | 83 |
| **27** | Bruhn, 2005, Fig. 4B | Saposin A | Danio rerio | 83 |
| **28** | Bruhn, 2005, Fig. 4B | Saposin A | Xenopus laevis | 83 |
| **29** | Bruhn, 2005, Fig. 4B | Saposin C | Homo sapiens | 80 |
| **30** | Bruhn, 2005, Fig. 4B | Saposin C | Bos taurus | 79 |
| **31** | Bruhn, 2005, Fig. 4B | Saposin C | Mus musculus | 79 |
| **32** | Bruhn, 2005, Fig. 4B | Saposin C | Gallus | 80 |
| **33** | Bruhn, 2005, Fig. 4B | Saposin C | Xenopus laevis | 79 |
| **34** | Bruhn, 2005, Fig. 4B | Saposin C | Danio rerio | 79 |
| **35** | Bruhn, 2005, Fig. 4B | Saposin D | Homo sapiens | 81 |
| **36** | Bruhn, 2005, Fig. 4B | Saposin D | Bos taurus | 81 |
| **37** | Bruhn, 2005, Fig. 4B | Saposin D | Mus musculus | 81 |
| **38** | Bruhn, 2005, Fig. 4B | Saposin D | Gallus | 81 |
| **39** | Bruhn, 2005, Fig. 4B | Saposin D | Danio rerio | 81 |
| **40** | Bruhn, 2005, Fig. 4B | Saposin D | Xenopus laevis | 81 |
| **41** | Bruhn, 2005, Fig. 4B | Saposin B | Homo sapiens | 79 |
| **42** | Bruhn, 2005, Fig. 4B | Saposin B | Bos taurus | 79 |
| **43** | Bruhn, 2005, Fig. 4B | Saposin B | Gallus gallus | 83 |
| **44** | Bruhn, 2005, Fig. 4B | Saposin B | Mus musculus | 83 |
| **45** | Bruhn, 2005, Fig. 4B | Saposin B | Danio rerio | 79 |
| **46** | Bruhn, 2005, Fig. 4B | Saposin B | Xenopus laevis | 79 |
| **47** | XP_003552303.1 | Saposin | Glycine max | 74 |
| **48** | XP_004506439.1 | Saposin | Cicer arietinum | 74 |
| **49** | XP_008388327.1 | Saposin | Malus x domestica | 73 |
| **50** | | Saposin A | Vicugna pacos | 81 |
| **51** | | Saposin A | Panthera tigris altaica | 84 |

A SAPLIP used according to the invention may also be a polypeptide comprising the saposin-fold as part of a multidomain protein. This is for example the case in acid sphingomyelinase (from Homo sapiens, Caenorhabditis elegans, Ciona intestinalis, Anopheles, Drosophila, Mus musculus or Rattus norvegicus); GDSL (Gly-Asp-Ser-Leu) lipase such as acyloxy hydrolase (from Homo sapiens or Rattus norvegicus); Countin (from Dictyostelium discoideum); J3-crystallin (from Tripedalia cystophora) and Plant aspartic proteases (from Viridiplantae). A further SAPLIP used according to the invention can be bacteriocin AS-48. Bacteriocin AS-48 displays antimicrobial activity, is able to bind lipids and possesses the same fold as SAPLIP family members. Bacteriocin AS-48, however, is devoid of any disulphide bridges.

Whereas in the following, the invention is described in more detail for saposin A or a derivative from thereof, and whereas saposin A or a derivative from thereof is a preferred embodiment, which is particularly suitable to form the particles for use in the present invention, the invention shall not be limited thereto. Due to the high degree of structural und functional conservation among SAPLIPs, the features and advantages of certain embodiments with saposin A as saposin-like protein also apply to other embodiments using other SAPLIPs or derivative forms thereof.

According to a one embodiment, the saposin-like protein or SAPLIP is saposin A, saposin B, saposin C or saposin D. In another embodiment, the SAPLIP is saposin A, saposin B or saposin D. Preferably, the saposin A, saposin B, saposin C or saposin D is from Homo sapiens, Equus caballus, Bos taurus, Mus musculus, Oryctolagus cuniculus, Rattus norvegicus or Xenopus laevis. In a more preferred embodiment, the SAPLIP is of human origin (i.e. a Homo sapiens SAPLIP). Such saposin-like proteins are particularly suitable to form the particles for use in the present invention.

In one embodiment, the SAPLIP is saposin A, preferably saposin A from Homo sapiens, Equus caballus, Bos taurus, Mus musculus, Oryctolagus cuniculus, Rattus norvegicus or Xenopus laevis. Most preferably, the SAPLIP is human saposin A having the amino acid sequence of SEQ ID NO. 1. The expression, purification and crystallization of Saposin A as LDAO-detergent complex is for example, described in PNAS, Vol. 109, No.8 (2012) 2908-2912 (Popovic et al.). These embodiments of saposin A are particularly suitable to form the particles for use in the present invention.

In a further embodiment, the saposin-like protein is a derivative of a SAPLIP, in particular a polypeptide comprising an amino acid sequence with at least 20,25, 30, 40, 50 or 60 %, preferably at least 75 % identity to the underlying full length SAPLIP. Preferably, the saposin-like protein comprises a sequence having an identity with the underlying full length sequence of a SAPLIP of at least 80 %, 85 %, 90 % or 95 %. Such derivatives of saposin-like proteins are particularly suitable to form the particles for use in the present invention.

The term "sequence identity" as used herein refers to a degree of identity between proteins that can be calculated by optimal alignment of the sequences using a scoring matrix such as the Blosum62 matrix described in Henikoff S. and Henikoff JG., P. N. A. S. USA 1992, 89: 10915-10919. Calculation of the percentage identity and optimal alignment of two sequences using the Blosum62 similarity matrix and the algorithm of Needleman and Wunsch (J. Mol. Biol. 1970, 48: 443-453) can be performed using the GAP program of the Genetics Computer Group (GCG, Madison, WI, USA) using the default parameters of the program. The EMBL-online tool "EMBOSS Stretcher" (http://www.ebi.ac.uk/Tools/psa/emboss stretcher/) can for example be used to determine the sequence identity.

In one embodiment, the saposin-like protein is saposin A, saposin B, saposin C, saposin D or a derivative form thereof, which is capable of forming saposin lipoprotein particles. Such derivatives of saposin-like proteins are particularly suitable to form the particles for use in the present invention. The skilled person can easily assess whether saposin lipoprotein particles have formed, e.g., by size exclusion chromatography (SEC).

In another embodiment, the derivative form of the saposin-like protein is
i. a protein having at least 20 %, preferably 25 %, more preferably 30 %, most preferably 35 % sequence identity to the full length sequence of any sequence selected from SEQ ID NO. 1-51, preferably selected from SEQ ID NO. 1, 2, 3, 4, 5, or 6; in particular wherein said protein is amphipathic, forms at least one alpha helix, and is capable of self-assembling together with lipids into a particle according to the invention; and/or
ii. a protein comprising a sequence selected from SEQ ID NO. 1-51, preferably selected from SEQ ID NO. 1, 2, 3, 4, 5, or 6,
   - in which 1 to 40 amino acids, preferably 1 to 35, more preferably 1to 30, most preferably 1 to 25 have been deleted and/or substituted; and/or
   - in which additional amino acids have been inserted or added.

The term "deletion" as used herein refers to the removal of one or more amino acid residues from a reference sequence. The term "insertion" as used herein refers to the insertion of one or more amino acid residues into a reference sequence. The term "addition" as used herein refers to the N- or C-terminal addition of one or more amino acid residues to a reference sequence. The term "substitution" as used herein refers to the exchange of an amino acid residue located at a certain position in the sequence to a different one.

According to another embodiment, the lipid binding polypeptide is a derivative of saposin A that comprises one or more fragments of SEQ ID NO. 1. Preferred fragments are helices a1, a2, a3 and a4 of saposin A, wherein helix a1 is formed by the following continuous stretch of amino acids: "SLPCDICKDVVTAAGDMLK"; helix a2 is formed by the following continuous stretch of amino acids: "ATEEEILVYLEKTCDWL"; helix a3 is formed by the following continuous stretch of amino acids: "PNMSASCKEIVDSYLPVILDIIKGEMS"; and helix a4 is formed by the following continuous stretch of amino acids: "PGEVCSAL". According to another embodiment, the derivative of the saposin-like protein is a derivative of saposin A comprising a sequence selected from helices a1, a2, a3, a4 of saposin A and combinations thereof. Preferably, the derivative of the saposin-like protein is a derivative of saposin A wherein the polypeptide comprises the sequences of helices a1, a2 and a3 of saposin A, wherein optionally helices a1, a2, a3, a4 have one or more amino acid deletions, additions, insertions and/or substitutions in the amino acid sequence.

In one embodiment, the derivative form of the saposin-like protein is a protein having at least 20 %, preferably 25 %, more preferably 30 %, most preferably 35 % sequence identity to the full length sequence of a sequence selected from SEQ ID NO. 1-51, preferably selected from SEQ ID NO. 1, 2, 3, 4, 5 or 6, is amphipathic, forms at least one alpha helix, and is capable of self-assembling together with lipids into a particle according to the invention. Such derivatives of saposin-like proteins are particularly suitable to form the particles for use in the present invention. As used herein, the term "amphipathic" refers to polypeptides or molecules having both hydrophilic and hydrophobic regions.

In one embodiment, the derivative form of the saposin-like protein is amphipathic, forms at least one alpha helix, has six cysteine residues corresponding to the six cysteines in the SAPLIP member saposin A, and is capable of self-assembling together with lipids into a particle according to the invention. Regarding the positions of the six cysteins, the positions as shown in the sequence comparison in Fig. 4A and 4B of Bruhn (2005), Biochem J 389 (15): 249-257, which Figures are hereby specifically incorporated by reference and reproduced as Figure 8a, 8b herein, apply.

In a further embodiment, the saposin-like protein or the derivative form thereof includes one or more non-natural amino acids, amino acid analogs, or peptidomimetic structures, in which the peptide bond is replaced by a structure more resistant to metabolic degradation.

### The lipids

The employed particle also comprises lipids. These lipids are typically different, i.e. they are a mixture of different lipids. The terms "lipid" and "membrane lipid" are used interchangeably herein and are technical terms known to the skilled person. The term "lipid" as used herein refers to a substance of synthetic or biological origin that is soluble or partially soluble in organic solvents. The saposin lipoprotein particle typically comprises a mixture of lipids. The particles thus comprise at least 2, 3, 5, 10 or 20 different lipids.

In one embodiment, the lipids of the saposin lipoprotein particle for use in the invention are a lipid mixture that naturally occurs in archaea, viruses, prokaryotes, eukaryotic cells, tissues, organs, or eukaryotic cell or organelle membranes. For example, the lipids can be brain lipids, lipids from a heart extract, lipids from a liver extract, lipids from a yeast extract or lipids from an *E.coli* extract. Such lipid environments within the saposin lipoprotein particle have the advantage that they can mimic particularly well the natural membrane environment of a certain hydrophobic agent. In this way, the hydrophobic agent is particularly well stabilized in its native conformation.

The term "membrane" as used herein refers to any membranes comprising a layer of lipids. The term "cell membrane" refers to a biological membrane that separates the interior of cells from the outside environment. The complex structure and the plurality of components comprised in a cell membrane, such as membrane lipids and membrane proteins, are described in detail in Alberts et al., "The Cell", 4th edition, Macmillian Magazines Ltd, 2002 on pages 583 to 614 and in Campbell et al., "Biologie", 6th edition, Spektrum Verlag, 2003 on pages 163 to 177. The term "organelle membrane" refers to a membrane separating an organelle from other cellular compartments.

In one embodiment, the lipids contained in the saposin lipoprotein particles for use in the invention are selected from the group consisting of viral lipids, archaeal lipids, eukaryotic lipids, prokaryotic lipids, and mixtures thereof. In another embodiment, the lipids of the saposin lipoprotein particle are of synthetic origin. The term "synthetic lipids" as used herein refers to artificially designed and produced lipids as well as to natural lipids which have been chemically modified. In a further embodiment, the lipids in the saposin lipoprotein particle are mixture of natural lipids and synthetic lipids.

In another embodiment, the lipids contained in the saposin lipoprotein particles are selected from the group consisting of phospholipids, glycolipids, sterols, and mixtures thereof. These lipids are particularly suitable for use in the saposin lipoprotein particles since these lipid species are typically present in a eukaryotic cell or organelle membrane. Thus, these lipids mimic efficiently the natural membrane environment so that the hydrophobic agent can be optimally stabilized in its native conformation.

Phospholipids possess a polar part that dissolves in water (the phosphate "head"), and a hydrophobic non-polar part that does not ("the lipid tail"). These parts are connected by a glycerol moiety. In water, phospholipids can build a cluster with the heads facing the water molecules and the tails facing away from the water molecules. The fatty acid chains in phospholipids and glycolipids usually contain an even number of carbon atoms, typically between 16 and 20. Fatty acids with 16 or 18 carbon atoms are the most common. Fatty acids may be saturated or unsaturated. Double bonds contained therein are typically in the so called cis-configuration. Cis- and trans-isomerism is a term known to the skilled person, which is used in organic chemistry to refer to the stereoisomerism of functional groups next a double bond (cf. Cahn, R.S. & Ingold, C.K.; Prelog, V., "Specification of Molecular Chirality". Angewandte Chemie International Edition, 5 (4), p.385-415, 1966). Typical fatty acids that are present in cellular membranes as part of lipids are also described in Alberts et al., "The Cell", 4th edition, Macmillian Magazines Ltd, 2002 on pages 61 and 62. Further examples of phospholipids are phosphatidylcholine including POPC (1-palmitoyl-2-oleoyl-sn-glycero-3-phosphocholine), phosphatidylethanolamine, phosphatidylserine including POPS (1-palmitoyl-2-oleoyl-sn-glycero-3-phospho-L-serine), phosphatidylinositol, and sphingomyelin.

Glycolipids are lipids containing a carbohydrate attached by a glycosidic bond. Examples of glycolipids are glyceroglycolipids, galactolipids, sulfolipids, glycosphingolipids, glucocerebrosides, sulfatides, gangliosides, globosides, glycophosphosphingolipids, and glycophosphatidylinositols.

Sterols are a subgroup of steroids. Examples of sterols are cholesterol, campesterol, sitosterol, stigmasterol and ergosterol.

In one embodiment, the lipids contained in the saposin lipoprotein particle for use in the invention are eukaryotic lipids and/or prokaryotic lipids including phospholipids, glycosphingolipids, sterols, phosphatidylcholine, phosphatidylserine (PS), 2-oleoyl-1-pamlitoyl-sn-glycero-3-phosphocholine (POPC), 2-oleoyl-1-pamlitoyl-sn-glycero-3-glycerol (POPG), 2-oleoyl-1-pamlitoyl-sn-glycero-3-phosphoethanolamine (POPE), diacylglycerol, cholesterol, sphingomyelin, galactosylceramide, gangliosides, phosphatidylinositoles, sulphogalactoceramides and combinations thereof.

In another embodiment, the lipids of the saposin lipoprotein particle for use in the invention comprise or consist of phospholipids. Examples of suitable phospholipids include, but are not limited to, DMPC, DMPG, POPC, dipalmitoylphosphatidylcholine (DPPC), dipalmitoylphosphatidylserine (DPPS), cardiolipin, dipalmitoylphosphatidylglycerol (DPPG), distearoylphosphatidylglycerol (DSPG), egg yolk phosphatidylcholine (egg PC), soy bean phosphatidylcholine, phosphatidylinositol, phosphatidic acid, sphingomyelin, and cationic phospholipids.

In a further embodiment, the lipids of the saposin lipoprotein particle for use in the invention include a modified lipid. Such a modified lipid can comprise one or more functional moieties, such as a targeting moiety or a bioactive moiety. The targeting moiety can, for example, serve to purify or identify the particles. The bioactive moiety can be selected, for example, from a drug, a cytotoxic agent, an enzyme, a label, a fluorophore, a contrast agent and a radiolabel.

In one embodiment, the saposin lipoprotein particle for use in the invention includes apart from the lipids lipid-interacting components. These include detergents, fats, waxes, fat-soluble vitamins, monoglycerides, diglycerides, triglycerides, fatty acids. The lipid-interacting components are, however, not limited thereto.

The term "detergent" as used herein is a technical term known to the skilled person and does not comprise lipids. While many detergents have a similar amphiphilic structure as lipids, i.e. a polar hydrophilic head group and a nonpolar hydrophobic tail, detergents differ from lipids, e.g., in the shape of the monomers and in the type of aggregates formed in solution. Lipids are generally substantially cylindrical in structure; the volume occupied by the hydrophobic tail is similar to the volume occupied by the polar head group. Detergent monomers are generally more cone-shaped; the volume occupied by the hydrophobic tail is smaller than the volume occupied by the polar head group. Detergents tend to aggregate into spherical or ellipsoid micelles that are water soluble without forming bilayer structures in the absence of lipids (cf. handbook "Detergents and their uses in membrane protein science" from Anatrace, www.anatrace.com).

Detergents employed in the saposin lipoprotein particles can be anionic deteregents, cationic deteregents, non-ionic detergents, zwitterionic detergents and mixtures thereof.

Typical anionic detergents are alkylbenzenesulfonates. The alkylbenzene portion of these anions is lipophilic and the sulfonate portion is hydrophilic. Such anionic detergents can, e.g., comprise branched alkyl groups or linear alkyl groups. Examples of suitable anionic detergents are bile acids including deoxycholic acid (DOC), alkylbenzenesulfonates including branched sodium dodecylbenzenesulfonate, linear sodium dodecylbenzenesulfonate, and mixtures thereof.

Cationic detergents are similar in structure to the anionic ones, but comprise as charged portion a cationic portion instead of an anionic portion. A suitable cationic portion is, e.g., a quaternary ammonium.

Non-ionic detergents are characterized by their uncharged, hydrophilic headgroups. Typical non-ionic detergents are, e.g., based on polyoxyethylene or a glycoside. Common examples include Tween, Triton, and the Brij series. Glycosides have a sugar as their uncharged hydrophilic headgroup. Examples include octyl thioglucoside and maltosides. The glycoside headgroup can also be of high-molecular-weight such as for Saponins. Saponins consist of a sugar moiety linked to a triterpene or steroid aglycone. Depending on the non-saccharide portion of the Saponin molecule, the Saponins can be divided into triterpene glycosides, steroid glycosides and steroid alkaloid glycosides. Examples of non-ionic detergents that can be contained in the saposin lipoprotein particles include saponins, e.g., Aescin, Bacopaside, Bacoside, Chaconine, Charantin, Digitonin, Glycyrrhizin, Ginsenoside, Holothurin, Protodioscin, Solanine, and mixtures thereof. Synthetic detergents structurally similar to Saponins such as glycol-diosgenin can also be used in the saposin lipoprotein particles of the present invention. Further examples of suitable non-ionic detergents that can be present in the saposin lipoprotein particles of the present invention are alkyl glycosides such as short, medium or longer chain alkyl maltosides including n-Dodecyl-β-maltoside (DDM), Decanoyl-N-hydroxyethylglucamide (HEGA) and n-Decanoyl-N-methyl-D-glucamide (MEGA).

Zwitterionic detergents possess a net zero charge arising from the presence of equal numbers of adversely charged chemical groups, i.e. the amount of negative charges equals the number of positive charges so that the overall charge is zero. Examples include Fos-Cholines, CHAPS/CHAPSO, and lauryl-dimethyl amine-oxides (LDAO).

In one embodiment, the detergent present in the saposin lipoprotein particles for use in the present invention is selected from the group consisting of alkylbenzenesulfonates, bile acids, cationic detergents, non-ionic detergents and zwitterionic detergents including lauryl-dimethyl amine-oxides (LDAO), Fos-Cholines, CHAPS/CHAPSO, saponins such as Digitonin, glycol-diosgenin, alkyl glycosides such as short, medium or longer chain alkyl maltosides including n-Dodecyl β-D-maltoside, glucosides, maltose-neopentyl glycol (MNG) amphiphiles, amphiphilic polymers (amphipols), styrene maleic acid co-polymer (SMA), macrocycle or cyclic oligomers based on a hydroxyalkylation product of a phenol and an aldehyde (Calixarene), and combinations thereof.

The amount of detergent in the saposin lipoprotein particles can be low to undetectable. In one embodiment, the saposin lipoprotein particle does not comprise any substantial amounts of detergent, in particular less than 0.1 wt.-%, preferably less than 0.01 wt.-%, particularly preferred less than 0.001 wt.-% detergent based on the weight of the particle. The amount of detergent (or other components) present in the particles can be determined, for example, by mass spectrometry.

In one embodiment, the lipids in the saposin lipoprotein particle are lipid bilayer forming lipids and/or biocompatible lipids. Lipids from archaeal membranes can also form monolayers.

The term "biocompatible" as used herein refers to biological compatible lipids which are not producing a toxic, injurious, or immunological response in a living tissue.

As used herein, "bilayer-forming lipid" refers to a lipid that is capable of forming a lipid bilayer with a hydrophobic interior and a hydrophilic exterior. Bilayer-forming lipids include, but are not limited to, phospholipids, sphingolipids, glycolipids, alkylphospholipids, ether lipids, and plasmalogens.

Particles may also include lipids that are not bilayer-forming lipids. Such lipids include, but are not limited to, cholesterol, cardiolipin, phosphatidylethanolamine (this lipid may form bilayers under certain circumstances), oxysterols, plant sterols, ergosterol, sitosterol, cationic lipids, cerebrosides, sphingosine, ceramide, diacylglycerol, monoacrylglycerol, triacylglycerol, gangliosides, ether lipids, alkylphospholipids, plasmalogens, prostaglandins, and lysophospholipids.

### The hydrophobic agent

The employed particle further comprises one or more hydrophobic agents.

The term "hydrophobic agent" as used herein refers to an agent which does not fully remain soluble in water and/or which tends to aggregate when present in an aqueous phase. Hydrophobic agents as defined herein thus generally comprise at least one hydrophobic (e.g., lipophilic) region capable of associating with or integrating into the hydrophobic portion of a lipid bilayer. The hydrophobic agent can also be a chimeric molecule, wherein a hydrophobic (e.g., lipophilic) moiety, module or compound capable of associating with or integrating into the hydrophobic portion of a lipid bilayer has been attached to a hydrophilic moiety, module or compound. An example for such a chimeric molecule is a lipid- or fatty acid-coupled molecule.

The hydrophobic agent is different from the lipids comprised in the saposin lipoprotein particle. This means that if the hydrophobic agent is itself a lipid or a modified lipid, the majority of lipids comprised in the particle (i.e. greater 50 mol-% based on the total amount of lipids present in the particle) should be different from the lipid that forms the hydrophobic agent. In one embodiment, the hydrophobic agent is not a lipid.

The hydrophobic agent is also different from the biologically active agent (e.g., drug molecule), which is optionally contained in the saposin lipoprotein particle of the invention.

In one embodiment, the hydrophobic agent is a hydrophobic biomolecule, preferably a hydrophobic biomolecule selected from the group consisting of a membrane protein, an integral transmembrane protein, an integral monotopic membrane protein, a peripheral membrane protein, an amphitropic protein in a lipid-bound state, a lipid-anchored protein, a chimeric protein with a fused hydrophobic and/or transmembrane domain. Such hydrophobic agents can be solubilized and stabilized particularly efficiently by the saposin lipoprotein particles.

The term "membrane protein" as used herein does not encompass the saposin-like protein or a derivative form thereof.

The advantage of including hydrophobic agents into a saposin lipoprotein particle is that they become effectively solubilized in the stable structure of the particles so that the particles can be investigated, analysed and/or serve as delivery vehicle for the hydrophobic agents, e.g., for use in membrane protein research, in particular proteomics, medical applications such as pharmaceutical formulations, vaccine formulations, therapeutic treatments, prophylactic treatments, diagnostic treatments and cosmetic treatments, *in vitro* methods for studying the hydrophobic agent of the particles of the invention, and/or for the methods of the invention. Compared to classic means of solubilization via detergents or organic solvents, hydrophobic agents are efficiently maintained in their native conformation in the saposin lipoprotein particles. Thus, also the native functionalities of the hydrophobic agents such as catalytic activities or a ligand binding capabilities typically remain unaffected, when the hydrophobic agent is present in a saposin lipoprotein particle.

Integral membrane proteins are membrane proteins which are permanently bound to the lipid bilayer and usually require a detergent or apolar solvent to become displaced from the membrane.

Transmembrane proteins are integral membrane proteins that span across the membrane at least once. Examples of transmembrane proteins that can be incorporated into the saposin lipoprotein particles are G-protein coupled receptors (GPCRs), single-pass transmembrane proteins, transporters such as uniporters, symporters, antiporters, or channels such as ion channels.

Integral monotopic membrane proteins are permanently attached to the membrane only from one side and do not span across the membrane. This class includes membrane proteins that are tethered to the membrane via alpha-helical transmembrane anchors. Examples include cytochrome P450 oxidases and glycophorin A.

Peripheral membrane proteins are only temporarily or indirectly associated with the lipid bilayer or integral membrane proteins incorporated therein. Peripheral membrane proteins usually dissociate from membranes following treatment with a polar reagent with an elevated pH or high salt concentrations. Examples of peripheral membrane proteins include phospholipase A2 or C, lipoxygenases and cytochrome c.

Lipid-anchored proteins are bound to the lipid bilayer via lapidated amino acid residues, in particular prenylated or GPI-anchor modified amino acid residues. Examples include bacterial lipoproteins, G proteins and certain kinases.

Amphitropic proteins are proteins that exist in at least two conformational states: a lipid free, watersoluble state and a lipid bound state. Upon association with lipids, amphitropic proteins undergo a conformational change allowing them to become reversibly or irreversibly membrane-associated. Examples of amphitropic proteins are pore-forming toxins and antibacterial peptides.

### The first binding agent

The employed particle further comprises one or more first binding agents.

The first binding agent is capable of binding to the saposin-like protein or a derivative form thereof and/or to one or more particle lipids. The first binding agent can bind to a natural epitope in the saposin-like protein or the derivative form thereof and/or to a natural epitope in one or more of the particle lipids. Alternatively, the first binding agent can bind to an artificial epitope that was incorporated in the saposin-like protein, the derivative form thereof and/or one or more particle lipids. Such an artificial epitope can be, for example, an affinity tag such as a peptide tag (including, but not limited to, a V5-tag, a Myc-tag, or an HA-tag) or a protein tag (including, but not limited to, Gluthathion-S-transferase (GST), or green fluorescent protein). Other suitable affinity tags are known to the skilled person.

The term "affinity tag" as used herein is given its ordinary meaning in the art. An affinity tag thus is any molecular structure that can be attached to a target molecule to facilitate binding, purification and/or detection of the target molecule. The skilled person also knows suitable methods to attach an affinity tag to a target molecule.

In one embodiment, the first binding agent is capable of binding only to particle-contained saposin-like protein or a particle-contained derivative form thereof and substantially not to the corresponding free, i.e. non-particle-contained saposin-like protein or a non-particle-contained derivative form thereof. This has the advantage that when forming the particles, e.g., for use in step a) of the method of the invention, the first binding agent is not wasted and lost by the formation of complexes consisting of saposin-like protein and first binding agent. In one embodiment, "substantially not binding to free saposin-like protein or a derivative form thereof" means that the first binding agent binds more efficiently to particle-contained saposin-like protein or a particle-contained derivative form thereof than to non-particle-contained saposin-like protein or a non-particle-contained derivative form thereof as measured, e.g., in an ELISA assay or SPR. In another embodiment, "substantially not binding to free saposin-like protein or a derivative form thereof" means that no binding, i.e. no binding above the background level, to free non-particle-contained saposin-like protein or non-particle-contained derivative form thereof is detectable, e.g., in an ELISA assay or SPR.

In another embodiment, the first binding agent is selected from the group consisting of antibodies or antigen-binding portions thereof, polymers, affibodies, affilins, anticalins, atrimers, avimers, peptides, bicyclic peptides, Cys-knots, designed Ankyrin repeat proteins (DARPins), FN3 scaffolds, Fynomers, Kunitz domains, OBodies, monobodies, anticalins, affibodies, aptamers, megabodies and nanobodies. First binding agents of these types are particularly suitable to modify the properties of saposin lipoprotein particles comprising as core components the saposin-like protein or a derivative form thereof, lipids, and the hydrophobic agent. For example such first binding agents can effectively increase the particle size, enhance the rigidity of the particle, influence the orientation of the particle when present on a support, and/or alter (increase or decrease) the symmetry of the particle. Moreover, a first binding agent selected from this group can typically be designed in such a way that it binds specifically and/or with high affinity to its target. Thus, off-target binding of the first binding agent can substantially be avoided.

In a preferred embodiment, the first binding agent is an antibody or an antigen-binding portion thereof such as a polyclonal antibody, a monoclonal antibody, a multispecific antibody, a chimeric antibody, a single-chain antibody, a Fab fragment, a Fab' fragment, a F(ab')₂ fragment, an Fd fragment, an Fv, a single-chain Fv (scFv), a disulfide-linked Fv (sdFv), or a fragment comprising a VL or VH domain,.

The terms "antibody" and "immunoglobulin" are used interchangeably herein. An antibody or immunoglobulin comprises at least the constant domain of a heavy chain, and normally comprises at least the variable domains of a heavy chain and a light chain.

As used herein, the term "antibody" comprises various broad classes of polypeptides that can be distinguished biochemically. The heavy chains are classified as gamma, mu, alpha, delta, or epsilon (γ, µ, α, δ, ε) with some subclasses among them (e.g., γ1-γ4). It is the nature of this chain that determines the "class" of the antibody as IgG, IgM, IgA IgG, or IgE, respectively. The immunoglobulin subclasses (isotypes), e.g., IgG1, IgG2, IgG3, IgG4, are well characterized and are known to confer functional specialization. With regard to IgG, a standard immunoglobulin molecule comprises two identical light chain polypeptides of molecular weight approximately 23,000 Daltons, and two identical heavy chain polypeptides of molecular weight 53,000-70,000. The four chains are typically joined by disulfide bonds in a "Y" configuration. Light chains are classified as either kappa or lambda (κ, λ). Each heavy chain class can be bound with either a kappa or lambda light chain.

Both the light and heavy chains are divided into regions of structural and functional homology. In this regard, the variable domains of both the light (VL) and heavy (VH) chain portions determine antigen recognition and specificity. In contrast, the constant domains of the light chain (CL) and the heavy chain (CH1, CH2 or CH3) confer important biological properties such as secretion, Fc receptor binding and complement binding. By convention the numbering of the constant region domains increases as they become more distal from the antigen binding site or amino-terminus of the antibody.

The variable region allows the antibody to selectively recognize and specifically bind epitopes on antigens. In this regard, the VL domain and VH domain, specifically the subset of the complementarity determining regions (CDRs) within these variable domains, combine to form the variable region that defines a three dimensional antigen binding site. This quaternary antibody structure forms the antigen binding site present at the end of each arm of the Y. More specifically, the antigen binding site is defined by three CDRs on each of the VH and VL chains.

In naturally occurring antibodies, the six "complementarity determining regions" or "CDRs" present in each antigen binding domain are short, non-contiguous sequences of amino acids that are specifically positioned to form the antigen binding domain as the antibody assumes its three dimensional configuration. The remainder of the amino acids in the antigen binding domains, referred to as "framework" regions, show less inter-molecular variability. The framework regions largely adopt a β-sheet conformation and the CDRs form loops that connect, and in some cases form part of the β-sheet structure. Thus, framework regions act to form a scaffold that provides for positioning the CDRs in correct orientation by inter-chain, non-covalent interactions. The antigen binding domain formed by the positioned CDRs defines a surface complementary to the epitope on the antigen. This complementary surface promotes the non-covalent binding of the antibody to its cognate epitope. The amino acids comprising the CDRs and the framework regions, respectively, can be readily identified for any given heavy or light chain variable domain by the skilled person, since they have been precisely defined by Kabat et al. (1983) U.S. Dept. of Health and Human Services, "Sequences of Proteins of Immunological Interest" and Chothia and Lesk, J Mol. Biol. 796:901-917 (1987).

In a further embodiment, the first binding agent is grafted onto a scaffold protein without substantially perturbing the binding properties of the first binding agent to the saposin-like protein or a derivative form thereof and/or to one or more of the particle lipids. Suitable scaffold proteins are derived, e.g., from HopQ, YgjK, T4 lysozyme (T4L), maltose binding protein (BMP), green fluorescent protein (GFP), cytochrome b562 and glycogen synthase. The skilled person knows suitable methods to determine whether the first binding agent still binds to its target molecules. For example, it is clear to the skilled person that an ELISA assay could be used to assess the binding of the first binding agent to saposin lipoprotein particles.

"Without substantially perturbing the binding properties" as used herein means that the binding efficiency of the first binding agent including the scaffold protein is reduced less than 75%, more preferably less than 25%, and most preferably less than 5% in comparison to the same first binding agent without the scaffold protein. The binding efficiency can for example be assessed in an ELISA assay.

In one embodiment, the scaffold protein is a monomeric protein. In another embodiment, the scaffold protein is a protein with symmetry such as a multimeric scaffold or a protein forming virus-like particles. Thus, the protein scaffold of the first binding agent helps to modify the symmetry of the entire saposin lipoprotein particles. This can facilitate in step b) of the method of the invention the determination of a full or partial 2D or 3D structure of the saposin lipoprotein particle, in particular of the hydrophobic agent contained therein. This is in particular the case, if in step b) X-ray crystallography, cryo-EM or NMR, most preferably X-ray crystallography or cryo-EM, is used as structural biology method.

In one embodiment, the scaffold protein has at total molecular mass of at least 10 kDa. In this way, the first binding including the scaffold protein can efficiently increase the molecular weight of a saposin lipoprotein particles which can facilitate to determine a full or partial 2D or 3D structure of the hydrophobic agent contained therein by a structural biology method in step b) of the method of the invention, in particular when using X-ray crystallography, cryo-EM or NMR, most preferably X-ray crystallography or cryo-EM.

### The second binding agent

The employed particle optionally further comprises one or more second binding agents.

The second binding agent and the biologically active agent when both present in the particle of the invention are different from each other. The term biologically active agent or "drug molecule" as used herein in particular refers to molecules that perturb or inhibit by their binding the function of the hydrophobic agent and/or a lipid in the particle. In contrast, the term "second binding agent" refers to any kind of molecules capable of interacting with the hydrophobic agent and optionally lipids. For example, a second binding agent according to the invention can serve to stabilize the native conformation(s) of the particle-contained hydrophobic agent.

In one embodiment, the second binding agent is selected from the group consisting of an antibody or an antigen-binding portion thereof, polymers, affibodies, affilins, anticalins, atrimers, avimers, bicyclic peptides, Cys-knots, designed Ankyrin repeat proteins (DARPins), FN3 scaffolds, Fynomers, Kunitz domains, OBodies, monobodies, anticalins, affibodies, aptamers, megapbides and monobodies. Second binding agents of these kinds are particularly suitable to stabilize structural conformations and/or defined regions of a hydrophobic agent. Second binding agents can in particular also hold flexible conformations in place. Previous studies have shown that such second binding agents can support and facilitate structure determination of the hydrophobic agent to which they bind (see, e.g., Manglik, Aashish, Brian K. Kobilka, and Jan Steyaert. "Nanobodies to study G protein-coupled receptor structure and function." Annual review of pharmacology and toxicology 57 (2017): 19-37 and Staus, Dean P., et al. "Allosteric nanobodies reveal the dynamic range and diverse mechanisms of G-protein-coupled receptor activation." Nature 535.7612 (2016): 448-452).

In a preferred embodiment, the second binding agent is an antibody or an antigen-binding portion thereof such as a polyclonal antibody, a monoclonal antibody, a multispecific antibody, a chimeric antibody, a single-chain antibody, a Fab fragment, a Fab' fragment, a F(ab')₂ fragment, an Fd fragment, an Fv, a single-chain Fv (scFv), a disulfide-linked Fv (sdFv), a fragment comprising a VL or VH domain, or a nanobody.

Regarding the further embodiments related to second binding agents, the embodiments described in connection with the first binding agents apply *mutatis mutandis.*

In another embodiment, different second binding agents are used in the saposin lipoprotein particle. This has the advantage that different conformational regions or domains of the hydrophobic agent can be stabilized by the second binding agents which further facilitates the structure determination in step b) of the method of the invention.

In another embodiment, the saposin lipoprotein particle contains one or more first binding agents and one or more second binding agents. The generation of such a saposin lipoprotein particle in step a) of the method of the invention is particularly advantageous for the subsequent structure determination step b) since the particle properties, in particular its stability, is not only influenced on the particle scaffold level, but also at the level of the hydrophobic agent. The term "one or more" in connection with the first and second binding agents, respectively, means that multiple and optionally even different binding agents are used.

In one embodiment, the first and the second binding agent are part of one molecule or interact with each other. "Interacting with each other" in this context means that the first and the second binding agent can form a stable complex. A molecule combining both the first and the second binding agent can, e.g., be a bispecific antibody or a bispecific antigen-binding fragment thereof. Such embodiments have the advantage that the interaction of the first and second binding agents provides a further interaction which can stabilize the entire particle structure, improve the rigidity of the particle, influence the symmetry of the particle and/or the orientation of the particles when present on a support. Modification of these particle properties can facilitate the structure determination in step b) of the method of the invention.

### The biologically active agent or drug molecule

The employed particle optionally further comprises a biologically active agent as defined above, in particular one or more drug molecules. The one or more drug molecules can also be chemically different.

The biologically active agent is different from the first binding agent and the second binding agent. The terms "biologically active agent" or "drug molecule" as used herein thus do not comprise the first and second binding agent. A biologically active agent or drug molecule as used herein is a molecule of any type (e.g., a small organic molecule, a peptide, a DNA-based molecule, an RNA-based molecule, a sugar- containing molecule, a natural or synthetic molecule) capable of interacting at least with the hydrophobic agent and/or the particle lipids. Thus, the biologically active agent as defined herein is considered "biologically active" at least because of its binding to the hydrophobic agent and/or the particle lipids. Via this binding the biologically active agent can directly alter the function of the hydrophobic agent and/or the particle lipids. Instead or also in addition, the biologically active agent can exert a further biological function, e.g., the biologically active agent can act as a binding platform for other molecules which then provide for another biological function. A "biologically active agent" or "drug molecule" as used herein can in particular be a small molecule compound with a molecular weight lower than < 900 daltons.

Providing a saposin lipoprotein particle additionally comprising one or more biologically active agents or drug molecules has the advantage that in the structure determination step b) of the method of the invention a binding site of the drug molecule can be determined or studied on the target hydrophobic agent. This identified binding site can then be used to better understand the binding behavior or to design further drug molecules which fit even better into the binding site. These are known as methods of structure-based drug design.

### Production of the particles

The saposin lipoprotein particles of step a) can essentially be produced by the methods described in WO 2014/095576 A1, WO 2018/033657 A1 and WO 2020/212423 A1. The additional particle components of the present inventions (the first binding agent, the second binding agent and the drug molecule) are not essential for particle formation can be added before, during and/or after particle assembly. Particle assembly typically takes place spontaneously upon contacting a saposin-like protein or a derivative form thereof with lipids and a hydrophobic agent.

The methods for producing saposin lipoprotein particles disclosed in WO 2014/095576 A1, WO 2018/033657 A1 and WO 2020/212423 A1, in particular the methods as described in the claims and the Examples of these applications, are included by reference in their entirety herein.

In brief, saposin lipoprotein particles can, e.g., be prepared by a process comprising the steps of
a) contacting the lipid binding polypeptide in form of the saposin-like protein or the derivative form thereof with solubilized lipids and a solubilized hydrophobic agent in a liquid environment; b) allowing for the self-assembly of the particle at a pH of from 5.0 to 10.0 (cf. WO 2014/095576 A1).

It is also possible to prepare saposin lipoprotein particles, in particular a library of saposin lipoprotein particles, wherein the particles comprise membrane components from a cell or an organelle membrane, by a method comprising the steps of a) providing a mixture of crude membrane vesicles obtained from a cell or an organelle membrane; b) contacting the mixture of step a) with the lipid binding polypeptide in a liquid environment; c) allowing for self-assembly of the particles (cf. WO 2018/033657 A1).

Further, it is possible to make saposin lipoprotein particles, wherein the produced saposin lipoprotein particles comprise a saposin-like protein, lipids, and optionally, a hydrophobic agent, wherein the hydrophobic agent is different from the lipids
(I) wherein the process comprises the following steps:
   a) providing the lipids, and optionally the hydrophobic agent;
   b.1) contacting the saposin-like protein with a support that is capable of selectively binding the saposin-like protein to the support in a liquid environment;
   c.1) contacting the support-bound saposin-like protein with the lipids and, optionally, the hydrophobic agent, to allow for the self-assembly of the saposin lipoprotein particle on the support;
   d) optionally eluting the support-bound saposin lipoprotein particle; or
(II) wherein alternatively the process comprises the following steps:
   a) providing the hydrophobic agent and the lipids;
   b.2) contacting the hydrophobic agent with a support that is capable of selectively binding the hydrophobic agent to the support;
   c.2) contacting the support-bound hydrophobic agent with the saposin-like protein to allow for the self-assembly of the saposin lipoprotein particle on the support;
   d) optionally eluting the support-bound saposin lipoprotein particle.

### Step b)

In step b) of the method of the invention the full or partial 2D or 3D structure of the particle provided in step a) is determined using a structural biology method.

In one embodiment, the structural biology method of step b) is selected from the group consisting of nuclear magnetic resonance spectroscopy (NMR), X-ray crystallography, small-angle X-ray scattering (SAXS), surface-plasmon resonance (SPR), and electron microscopy (EM). Good results were achieved with the structural biology method being negative-stain electron microscopy and/or cyro-electron microscopy (cryo-EM). How these methods are employed to determine the structure of protein complexes or parts thereof is well-known to the skilled person.

In a preferred embodiment, the structural biology method employed in step b) is X-ray crystallography, NMR or electron microscopy (EM), in particular negative-stain and/or cryo-EM. The particles of the invention are particularly suitable for use in these methods since the first binding agent and optionally the second binding agent increase the particle size, enhance the rigidity of the particle, can be used to lock-in or at least unify to some extent the orientation of the particles, especially when present on a support, and/or alter (increase or decrease) the symmetry of the particle. These effects are particularly advantageous for structural biology.

For example, obtaining diffraction-quality crystals remains a major bottleneck in macromolecular X-ray crystallography. It has been found that the chances to obtain well-ordered crystals can be increased by minimizing the conformational heterogeneity in the crystallization target and by supplementing the surface of the target with further protein so that formation of primary contacts between the molecules in the lattice are facilitated. This can be achieved by adding the first, or optionally also the second, binding agent of the invention to the Salipro particles. For similar reasons, the particle modifications triggered by the first binding agent and optionally the second binding agent are also advantageous for cryo-EM. Cryo-EM has developed into a versatile technique for structural analysis of macromolecular complexes at atomic resolution. The analysis of smaller size, low symmetry, and highly flexible targets, in particular at high resolution, remains difficult by cryo-EM. Moreover, a preferred particle orientation due to surface properties of the target macromolecules that cause specific regions to preferentially adhere to the air-water interface or the support represent a recurring issue in cryo-EM. The use of the first binding agent and optionally the second binding agent in the saposin lipoprotein particles helps to decrease these performance barriers of the cryo-EM technique.

In one embodiment, the particle of step a) is at least 20 kDa, preferably at least 40 kDa, more preferably 60 kDa and most preferably at least 80 kDa in size. In this case, performance barriers which are typically higher for smaller sized molecular targets in structural biology methods, in particular X-ray crystallography and cryo-EM, are reduced.

The method of the invention allows to determine the full or partial 2D or 3D structure, preferably the full or partial 3D structure of the saposin lipoprotein particle provided in step a), at high resolution. Preferably, the full or partial 2D or 3D structure is determined at a resolution higher than 25 Å, preferably higher than 10 Å, more preferably higher than 10 Å, even more preferably higher than 5 Å and most preferably higher than 3 Å. Obtaining such high resolution structures have the advantage that they are particularly well suitable for structure-based drug designs.

### Use of the obtained 2D or 3D structure

The 2D or 3D structures determined according to the invention can be used advantageously for studying a biologically active agent or screening for such an agent, in particular a drug molecule that binds particularly well to the hydrophobic agent or to the hydrophobic agent and lipids. In case the biological agent is an antibody or an antigen-binding portion thereof, the determined 2 D or 3D structures can be used to determine the binding epitope of the antibody. This use if further described in connection with the following method of the invention which is particularly suitable for identifying, studying and further developing new drug candidates or identifying the binding sites of biologically active agents (e.g., small molecules or antibodies).

### Method for studying or identifying a biologically active agent

This method of the invention is suitable for studying or identifying a biologically active agent (in particular a drug candidate) that binds to a hydrophobic agent of interest. By its binding to the hydrophobic agent, the biologically active agent preferably perturbs or inhibits the native function of the hydrophobic agent. In the following, advantageous aspects of the method will be described with reference to a drug molecule as an example for a biologically active agent. Then the hydrophobic agent or the lipid(s) are the drug target. The explanations that follow are, however, intended to equally apply to other biologically active agents as defined above. Thus, the term "drug molecule" or "drug candidate" in this disclosure serves as a placeholder that can also be replaced by the broader term "biological active agent".

### Step a.

In step a. of the method of the invention one or more partial or full 2D or 3D structures, preferably one or more partial or full 3D structures, of a particle comprising
- a saposin-like protein or a derivative form thereof;
- lipids;
- a hydrophobic agent wherein the hydrophobic agent is different from the lipids;
- a first binding agent that is bound to the saposin-like protein or the derivative form thereof and/or the lipids;
- optionally a second binding agent that is bound to the hydrophobic agent; and
- optionally a drug molecule interacting with the hydrophobic agent and/or the lipids are obtained.

This can be achieved by the above-described method of the invention, i.e. the method of the invention for determining a full or partial 2D or 3D structure, preferably a full or partial 3D structure of
- a hydrophobic agent,
- a second binding agent interacting with the hydrophobic agent, and/or
- a binding site between
   - a biological active agent and a hydrophobic agent,
   - a biological active agent and lipids, or
   - a biological active agent, a hydrophobic agent and lipids.

The embodiments described in connection with the method of the invention for determining a full or partial 2D or 3D structure apply *mutatis mutandis* also to step a. of the method of the invention for identifying a new drug candidate.

### Step b.

In step b. of the method of the invention, the binding of a biological active agent such as a drug candidate to the hydrophobic agent is modeled using the one or more 2D or 3D structures of step a.

The term "modeling" means that a method analogous to "structure-based drug design" is performed using the one or more 2D or 3D structures of step a.

Structure-based drug design is a term known in the art and refers to the *in silico* modeling of a biologically active agent into the binding site of the target and design and optimizing, in an often iterative fashion, the structure of the biologically active agent to improve its fit and binding into the target binding site. This is done in particular with the goal of identifying a compound suitable for in vivo, in particular clinical testing, as in the case of a drug candidate.

As used herein, a "drug candidate" refers to a compound or biological molecule that is selected by the structure-based design method of step of step b. for binding to the target hydrophobic agent and/or lipids.

According to the present invention, the modeling of the binding of a drug candidate to the hydrophobic agent can include creating a new chemical compound or searching databases of libraries of known compounds (e.g., a compound listed in a computational screening database containing 3D structures of known compounds). The modeling can also include selecting a chemical compound based on a known function of this compound. Methods to synthesize suitable chemical compounds are known to those of skill in the art and depend on the structure of the chemical being synthesized.

Various methods for the structure-based drug design employed in step b. are known to the skilled person (see, e.g., Anderson, Amy C. "The process of structure-based drug design." Chemistry & biology 10.9 (2003): 787-797 and Ferreira, Leonardo G., et al. "Molecular docking and structure-based drug design strategies." Molecules 20.7 (2015): 13384-13421). These methods can be broadly classified as experimental (*in vitro, ex vivo* or *in vivo*) or computer-aided (at least partially *in silico*)*.* An example of an experimental method is a high-throughput screening with combinatorial chemistry, in which thousands of compounds are tested for biochemical effects. The computer-aided methods can be further classified into at least three categories: inspection, virtual screening, and *de novo* generation. During inspection, known molecules that bind a certain site of the target, such as substrates or cofactors in the case of enzymes, are modified to become inhibitors based on maximizing complementary interactions at the binding site. In the virtual screening method, databases of available small molecules are docked into the region of interest in silico and scored based on predicted interactions with the site. For the *de novo* generation method, small fragments of molecules, such as benzene rings, carbonyl groups, amino groups, etc., are positioned in the site, scored, and linked *in silico.* The final compounds, created *in silico* from the linked fragments, must then be synthesized in the laboratory. In particular with regard to the virtual screening method and the de novo generation method, the skilled person is aware of suitable drug design software.

### Step c.

In step c. of the method of the invention one or more drug candidates which are modeled to selectively bind to the hydrophobic agent in step b. are selected. The advantage of this step is that the further identification method is limited to and thus focused on the most promising candidates.

The selection of the drug candidates as performed in step c. is typically based on the performance of the drug candidate in the experimental or computer-aided structure-based drug design of step b. The selection criteria thus depend on the structure-based drug design method which was used in step b.

### Step d.

In the optional step d. of the method of the invention the selective binding of the selected one or more drug candidates of step c. to the hydrophobic agent in the particle of step a. is confirmed by contacting the particle with the selected one or more drug candidates and measuring the binding between the hydrophobic agent in the particle and the selected one or more drug candidates and/or the biological activity of the hydrophobic agent. A reduced, altered or inhibited activity of the hydrophobic agent is thereby indicative for the favorable binding characteristics of the drug candidate.

Methods of measuring the selective binding between two interaction partners and/or the biological activity of a target are known to the skilled person and include, e.g., SPR, immunoassays, kinase assays, flow cytometry, and phosphorylation assays.

### Particles

The invention also concerns a particle comprising
- a saposin-like protein or a derivative form thereof;
- lipids;
- a hydrophobic agent wherein the hydrophobic agent is different from the lipids;
- a first binding agent wherein the first binding agent is bound to
   - the saposin-like protein or the derivative form thereof and/or
   - the lipids;
- optionally
   - a second binding agent wherein the second binding agent is bound to the hydrophobic agent; and/or
   - a drug molecule interacting with the hydrophobic agent and/or the lipids.

The particles of the invention are particularly suitable for a variety of research and analytical purposes including membrane protein research, proteomics, medical applications such as vaccine formulations, therapeutic treatments, prophylactic treatments, diagnostic treatments and cosmetic treatments, *in vitro* methods for studying the hydrophobic agent of the particles of the invention, and/or for the methods of the invention. That the particles of the invention are particularly suitable for all these purposes is in particular due to the first binding agent. The first binding agent serves as a versatile "anchor" or "platform" to alter and adapt the particle properties. Since the saposin lipoprotein particles typically contain multiple saposin-like proteins and lipids, the particles of the invention usually comprise multiple first binding agents. In this way, the first binding agents of the saposin lipoprotein particle can modify particularly well the properties of the particles. Moreover, using first binding agents in this way, i.e. targeting the saposin-like proteins or lipids, and not the hydrophobic agent, has the advantage that the hydrophobic agent in the particle remains unmodified so that in particular its native function is not altered, perturbed or inhibited.

The presence of the first binding agent inherently increases the particle size and can also have an impact on the rigidity of the particle, the orientation of the particle when present on a support, and/or the symmetry of the particle, which supports structure determination of the particle components, in particular when using X-ray crystallography, cryo-EM or NMR, most preferably X-ray crystallography or cryo-EM, as structural biology method.

The embodiments described in connection with the methods of the invention above apply *mutatis mutandis* also to the particles and *vice versa.* This in particular applies to the particle components and the production of the particles.

In a further embodiment, the first binding agent for use in the particle of the invention can contain one or more further functional moieties apart from an antigen binding moiety, wherein the antigen binding moiety serves to bind the saposin-like protein or a derivative thereof and/or one or more of the particle lipids. The term "one or more" in connection with the functional moieties of the first binding agent means that multiple and optionally even chemically different further functional moieties can be present on the first binding agent.

In a further embodiment, the one or more further functional moieties have the same or a different function in comparison to the antigen binding moiety of the first binding agent, wherein the antigen binding moiety serves to bind the saposin-like protein or a derivative thereof and/or one or more of the particle lipids. In a further embodiment, the one or more further functional moieties have a different function in comparison to the antigen binding moiety of the first binding agent. Such embodiments have the advantage that the saposin lipoprotein particles of the invention are equipped with additional functionalities that can for example be particularly useful for research, medical or analytical purposes.

In one embodiment, the further functional moiety is not used to immobilize, bind, couple or retain the particle of the invention to a support.

The term "support" as used herein refers to any support material that is capable of immobilizing, binding, coupling or retaining a functional moiety of a first binding gent. The support, as used herein, can in particular be a "carrier", "carrier material", "stationary phase" or "solid support", e.g., in the form of a planar, curved, contorted, twisted and/or angular surface. The support as referred to herein is typically of artificial origin. Thus, the term "support" as used herein does not comprise completely natural support materials, e.g., the surface of a cell or organelle present *in vitro* or *in vivo.* The support can be functionalized to recognize functional moieties. Support as used herein in particular means a solid support with a macroscopically flat surface, e.g. a chip. In one embodiment the support is not in the form of beads or a resin.

In one embodiment, the further functional moiety is selected from the group consisting of a capture moiety, a multimerization moiety, a labeling moiety and combinations thereof. In a further embodiment, the further functional moiety is selected from the group consisting of a capture moiety, a multimerization moiety, a labeling moiety and combinations thereof, wherein the capture moiety interacts with a cargo molecule and the multimerization moiety is in complex with at least one multimer unit. A multimer unit can be any molecular unit capable of multimerizing with the multimerization moiety of the first binding agent. A multimer unit can for example be another saposin lipoprotein particle comprising a first binding agent with a multimerization moiety.

The term "capture moiety" as used herein is any molecular structure in the first binding agent that can specifically bind, fix, couple or retain a cargo molecule. A cargo molecule can be any type of molecule. For example a cargo molecule can be a drug molecule, a dye or a toxin. The particles of the invention can thus be used for targeting a cargo molecule loaded onto the first binding agent by the capturing moiety to *in vivo* target cells of interest based on specific interactions of the target cells with the hydrophobic agent contained in the particle. Due to the presence of typically several first binding agents and/or the presence of multiple capture moieties on the first binding agent, a high load of cargo molecule can be achieved per saposin lipoprotein particle. These particles can thus be particularly useful for medical applications.

The capture moiety of the support can enable selective binding of the cargo molecule by a variety of modes. For example: (i) capturing the cargo molecule by chemically coupling a binding moiety of the cargo molecule to the capture moiety of the first binding agent by a chemical bond, (ii) capturing the cargo molecule via affinity-based interactions between the capture moiety and a natural or engineered binding moiety in the cargo molecule, (iii) indirect binding of the cargo molecule by engineering the capture moiety in the support to bind to a bridging agent (e.g., a peptide epitope, substrate analog or ligand), which is selectively bound by a binding moiety in the cargo molecule, (iv) capturing the cargo molecule via hydrophobic interactions between the capture moiety of the first binding agent and a natural or engineered binding moiety in the cargo molecule and/or (iii) capturing the target molecule via charge-based interactions between the capture moiety and a natural or engineered binding moiety in the cargo molecule.

The term "binding moiety" as used herein in connection with the cargo molecule refers to the molecular structure in the cargo molecule that is bound, fixed, coupled or retained by the capture moiety of the first binding agent.

In one embodiment, the binding of the cargo molecule to the capture moiety of the first binding agent is achieved by chemically coupling the binding moiety of the cargo molecule to the capture moiety of the first binding agent by means of a chemical bond. This can be achieved by taking advantage of functional groups in the cargo molecule such as thiol-, amino-, hydroxyl-, aldehyde- or carboxyl groups. Suitable capture moieties of the first binding agent to react with such binding moieties of the cargo molecule are known to the skilled person. The skilled person is able to select a suitable capture moiety/binding moiety pair depending on the type of coupling to be achieved. Thiol groups in the cargo molecules can, for example, be reacted with maleimides, disulfides or iodacetamid used as capture moieties in the first binding agent or vice versa. Primary amines in cargo molecules can, for example, be reacted with succinimide esters (such as N-hydroxysuccinimide, sulfosuccinimide or other succinimidyl esters), imidoesters or isothiocyanates (such as phenylisothiocyanat) used as capture moieties in the first binding agent or vice versa. Carboxy groups of target molecules can, for example, be reacted with carbodiimides (such as 1-Ethyl-3-(3-dimethylaminopropyl)carbodiimid) used as capture moieties in the first binding agent or vice versa.

Depending on the chemistry chosen, the coupling of the cargo molecules to the first binding agent via formation of chemical bonds may be substantially irreversible, which may be beneficial for certain applications requiring particularly tight binding of the cargo molecule to the first binding agent. On the other hand, capture via affinity-based interactions, hydrophobic interactions or electrostatic interactions is usually reversible and has the advantage that the cargo molecule can be set free under certain conditions.

In another embodiment, the selective binding of the cargo molecule to the first binding agent is achieved by affinity-based interactions between the capture moiety of the first binding agent and a natural or engineered binding moiety in the cargo molecule. In this way, cargo molecules with a natural or engineered binding moiety are selectively bound by the first binding agents's capture moiety using affinity-based interactions.

Examples of natural capture moiety/binding moiety affinity-based pairs are lectins that bind to certain sugar moieties in the cargo molecule (e.g., if this is a glycosylated membrane protein). Post-translational modifications such as glycosylation, sulfation, palmitoylation, myristoylation, ubiquitination or SUMOylation can also serve as natural binding moieties in the cargo molecule according to the invention. Various other natural affinity-based interactions between a capture and a binding moiety are known to the skilled person.

Particular important examples of engineered affinity-based capture moiety/ binding moiety pairs come from the well-known area of affinity tags. Accordingly, in one embodiment the capture moiety of the first binding agent is an affinity tag or an affinity tag recognizing moiety. The respective partner moiety in the cargo molecule is in this case a specific counterpart partner moiety in form of a moiety recognizing the affinity tag with a high binding affinity or an affinity tag which is specifically bound by the affinity tag recognizing moiety of the first binding agent.

Affinity tags or affinity tag recognizing moieties can be attached to a first binding agent or a cargo molecule by any suitable method. In one embodiment, the affinity tag or the affinity tag recognizing moiety is attached to the first binding agent or the cargo molecule using genetic methods. In another embodiment, the affinity tag or the affinity tag recognizing moiety is attached to the first binding agent or the cargo molecule after the molecule has been produced (e.g., expressed or synthesized). For example biotin can be chemically coupled to a first binding agent or a cargo molecule to facilitate the binding of streptavidin which is coupled to the cargo molecule or the first binding agent.

Suitable affinity tags are known to the skilled person. Such affinity tags include, but are not limited to, GST (in glutathione/GST binding), biotin (in biotin/streptavidin binding), maltose (in maltose/maltose binding protein binding), epitope tag (in epitopte tag/antibody or antibody fragment interaction), protein A (in protein A/antibody or antibody fragment interaction), fluorescent protein (in fluorescent protein/antibody or antibody fragment interaction), Fc receptor (in Fc receptor/antibody or antibody fragment interaction) and a zinc finger (in zinc finger/nucleic acid interaction).

The term "multimerization moiety" as used herein refers to any molecular structure in the first binding that allows multimerization with one or more multimerization units. Such a multimerization unit consists of at least a multimerization moiety capable of binding to the multimerization moiety of the first binding agent. Suitable multimerization moieties are known to the skilled person.

In one embodiment, the multimerization moiety is selected from the group consisting of a moiety comprising or consisting of an amphipathic helix, a moiety comprising or consisting of a leucine zipper, a moiety comprising or consisting of a biotin acceptor peptides or protein, a moiety comprising or consisting of an Fc domain, and a moiety comprising or consisting of an oligomer forming peptide or protein. Such multimerization moieties are particularly useful in the particle of the invention for multimerization.

In another embodiment, the multimerization moiety is an immunoglobulin Fc domain. Non-limiting examples of immunoglobulin Fc domains are IgG, IgA, IgD, IgM, and IgE immunoglobulin Fc domains. In a further embodiment, the immunoglobulin Fc domain is an IgG1 immunoglobulin Fc domain. In another embodiment, the multimerization moiety is an immunoglobulin Fc domain that lacks effector functions as compared to a corresponding wild-type immunoglobulin Fc domain.

The term "labeling moiety" as used herein refers to any molecular structure in the first binding agent that facilitates isolation and/or detection of the saposin lipoprotein particles of the invention. A suitable molecular structure can be a defined structural element within the first binding agent or simply suitable amounts of certain isoptopes in the first binding agent such as radioactive isotopes (e.g., ²H, ¹³C, ³²P and ¹⁵N). In one embodiment, the labeling moiety is selected from the group consisting of a radioactive isotope, wherein the radioactive isotope is preferably ²H, ¹³C, ³²P and ¹⁵N, an affinity tag and combinations thereof. In a further embodiment, the labeling moiety is an affinity tag. Affinity tags have the advantage that they bind with high specificity to well-known, often commercially available interaction partners. As already explained above, suitable affinity tags and their individual advantages are known to the skilled person. For example, the affinity tag of the first binding agent can be a fluorescent molecule. Such a fluorescent molecule can be particularly useful to detect saposin lipoprotein particle in a spatio-temporal manner, e.g., when using these particles during B-cell sorting.

In particular, an affinity tag described herein in connection with the components of the particles can be selected from the group consisting of Albumin-binding protein (ABP), AU1 epitope, AU5 epitope, Bacteriophage T7 epitope (T7-tag), Bacteriophage V5 epitope (V5-tag), Biotin-carboxy carrier protein (BCCP), Bluetongue virus tag (B-tag), Calmodulin binding peptide (CBP), Chloramphenicol Acetyl Transferase (CAT), Cellulose binding domain (CBP), Chitin binding domain (CBD), Choline-binding domain (CBD), Dihydrofolate reductase (DHFR), E2 epitope, FLAG epitope, Galactose-binding protein (GBP), Green fluorescent protein (GFP) and fluorescent variants thereof, Glu-Glu (EE-tag), Glutathione S-transferase (GST), Human influenza hemagglutinin (HA), HaloTag^{®}, Histidine affinity tag (HAT), HSV epitope, Ketosteroid isomerase (KSI), KT3 epitope, LacZ, Luciferase, Maltose-binding protein (MBP), Myc epitope, NusA, PDZ domain, PDZ ligand, Polyarginine (Arg-tag), Polyaspartate (Asp-tag), Polycysteine (Cys-tag), Polyhistidine (His-tag), Polyphenylalanine (Phe-tag), Profinity eXact, Protein C, S1-tag, S-tag, Streptavadin-binding peptide (SBP), Staphylococcal protein A (Protein A), Staphylococcal protein G (Protein G), Strep-tag, Streptavadin, Small Ubiquitin-like Modifier (SUMO), Tandem Affinity Purification (TAP), Thioredoxin (Trx), TrpE, Ubiquitin, VSV-tag, Xpress-tag, Ty-tag and combinations thereof.

The terms "combinations thereof" or "mixtures thereof" as used herein in connection with lists means that different list members can be used together.

### Uses of the first binding agent

The invention further concerns certains uses of the first binding agent. The first binding agent capable of binding to the saposin-like protein or a derivative form thereof and/or to one or more particle lipids can be used in a saposin lipoprotein particle for increasing the particle size, influencing the orientation of the particle when present on a solid support, and/or enhancing the rigidity of the particle to determine the 2D or 3D structure, preferably the 3D structure, of a hydrophobic agent contained in the particle. As described above in connection with the methods of the invention, altering these particle properties can significantly help to obtain a full or partial 2D or 3D structure of the saposin lipoprotein particle, in particular when using X-ray crystallography, cryo-EM or NMR, most preferably X-ray crystallography or cryo-EM, as structural biology method.

Further, the first binding agent can be used according to the invention in a saposin lipoprotein particle for multimerizing, capturing and/or labeling the particles. As described above in connection with the particles of the invention, the saposin lipoprotein particles can be further functionalized in this way without altering, perturbing and/or inhibiting the hydrophobic agent contained in the particle. The use of the first binding agent in such a way also offers the possibility to particularly effectively functionalizing the saposin lipoprotein particles since typically multiple first binding agents can bind per particle due to the presence of multiple saposin-like proteins, derivative forms thereof and/or lipids in a single particle. Further, first binding agents can be used for saposin lipoprotein particles independent of the hydrophobic agent contained therein since the first binding agent binds to the particle scaffold. Thus, first binding agents for functionalizing saposin lipoprotein particles are "universally applicable" to any kind of saposin lipoprotein particle independent of the hydrophobic agent contained therein. Such particles are therefore particularly useful for a variety of research, medical and analytical purposes including membrane protein research, proteomics, medical applications such as pharmaceutical compositions, vaccine formulations, therapeutic treatments, prophylactic treatments, diagnostic treatments and cosmetic treatments, *in vitro* methods for studying the hydrophobic agent of the particles of the invention, and/or for the methods of the invention.

The term "multimerizing" as used herein means "to form a complex containing the same or different multimeric units". Multimerization can be achieved by making use of multimerization moieties. Regarding the use of the multimerization moieties, the embodiments described in connection with the particles of the invention apply *mutatis mutandis.*

The term "capturing" as used herein means in particular "to capture a cargo molecule". Capturing can be achieved by making use of capture moieties. Regarding the use of the capture moieties, the embodiments described in connection with the particles of the invention apply *mutatis mutandis.*

The term "labeling" as used herein means in particular "to facilitate isolation and/or detection". Labeling can be achieved by making use of labeling moiety. Regarding the use of the labeling moieties, the embodiments described in connection with the particles of the invention apply *mutatis mutandis.*

### Further uses of the particles

The invention also concerns the use of the saposin lipoprotein particles of the invention in pharmaceutical composition, in particular a vaccine formulation, a therapeutic or prophylactic method, in particular in a method to prevent, treat or lessen the severity of a disease, a diagnostic method, or a cosmetic treatment.

In one embodiment, the saposin lipoprotein particles of the invention are used in a pharmaceutical composition for delivering one or more hydrophobic agents, drugs and/or lipids to an individual in need thereof. In a further embodiment, the pharmaceutical composition containing the saposin lipoprotein particles of the invention can optionally comprise a (further) pharmaceutically acceptable vehicle, carrier or adjuvant.

In case, the saposin lipoprotein particles are used in a pharmaceutical composition, the individual components of the particles and the pharmaceutical composition are pharmaceutically acceptable. As used herein, the term "pharmaceutically acceptable" refers to components, compounds or agents that are suitable for use in contact with the tissues of humans and/or animals without undue toxicity, irritation, allergic response and the like. "Pharmaceutically acceptable components" are also characterized by a reasonable benefit/risk ratio.

The pharmaceutical compositions containing saposin lipoprotein particles can be administered to humans and non-human animals orally, rectally, parenterally, intracisternally, intravaginally, intraperitoneally, topically (as by powders, ointments, or drops), buccally (as an aerosol, an oral spray or nasal spray), or the like. The choice of the administration route typically depends on the type and severity of the disease being treated.

In another embodiment, the saposin lipoprotein particles are used in a vaccine formulation. Many pathogenic antigens that could be potent antigens in vaccines are exposed on the surface and/or comprised in the outer cell membrane of a eukaryotic pathogen, a prokaryotic pathogen or a disease cell (e.g., a cancer cell) in a patient. Such antigens can effectively be incorporated into the particles of the invention. Thus, the saposin lipoprotein particles can be used as antigen-presenting delivery vehicles in vaccine formulations.

In a further embodiment, the saposin lipoprotein particles are used in a therapeutic or prophylactic method. These are in particular methods to prevent, treat or lessen the severity of a disease.

The term "disease" refers to any deviation from the normal health of a subject and includes a state when disease symptoms are present, as well as conditions in which a deviation (e.g., infection, gene mutation, genetic defect, etc.) has occurred, but symptoms are not yet manifested.

In one embodiment, the saposin lipoprotein particles are used in a diagnostic method. A "diagnostic method" as used herein typically refers to treatments with a curative purpose comprising the following phases: (i) the examination phase, involving the collection of data, (ii) the comparison of these data with standard values, (iii) the finding of any significant deviation, i.e. a symptom, during the comparison, (iv) the attribution of the deviation to a particular clinical picture, i.e. the deductive medical or veterinary decision phase.

In a further embodiment, the saposin lipoprotein particles are used in a cosmetic treatment. A "cosmetic treatment" as used herein is an invasive or non-invasive non-curative treatment with no substantial health risk for the treated subject.

### BRIEF DESCRIPTION OF THE DRAWINGS

The invention is further described herein with reference to the Figures, which depict certain embodiments of the invention. The invention, however, is as defined in the claims and as generally described herein. The invention should not be limited to the embodiments shown for illustrative purposes in the Figures below.

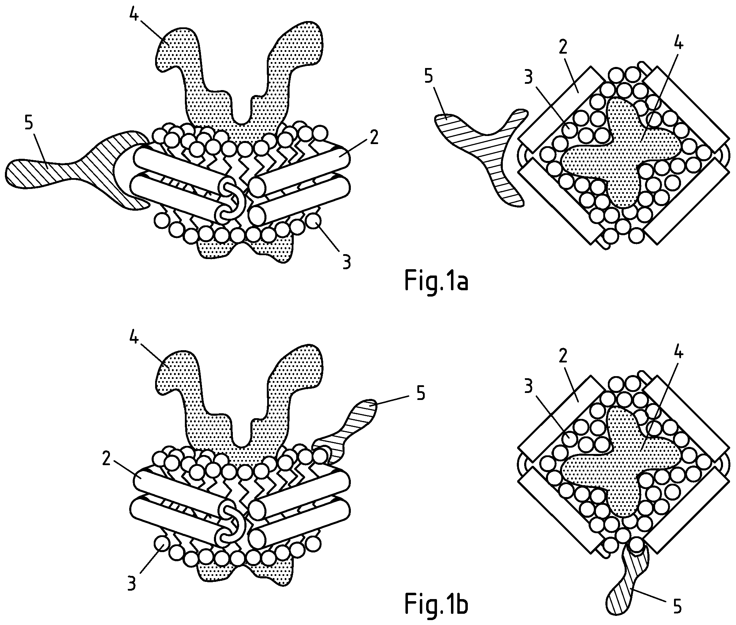

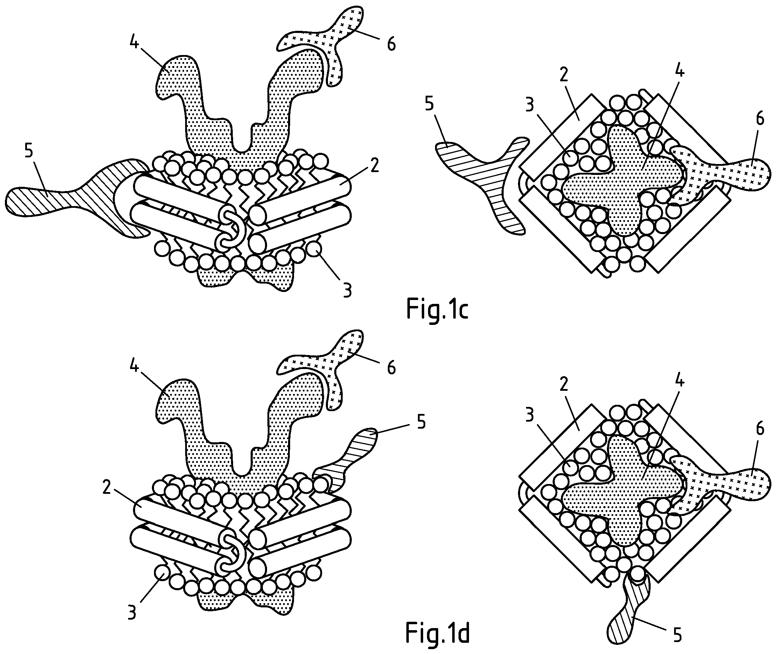

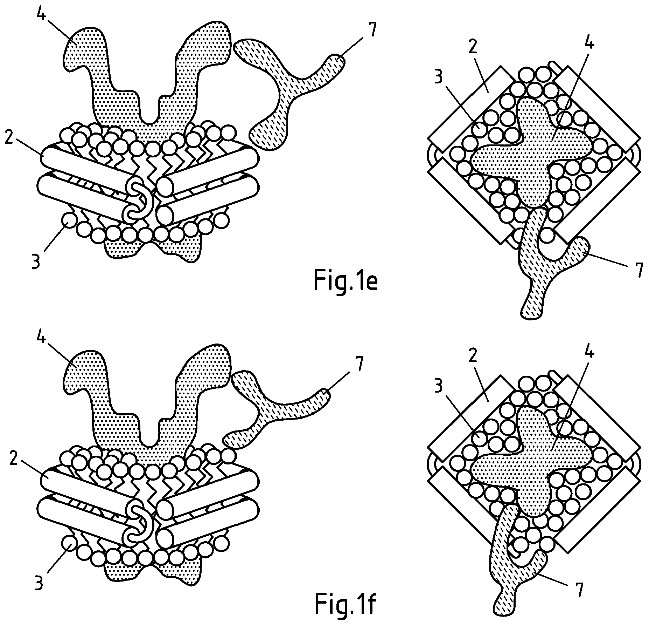

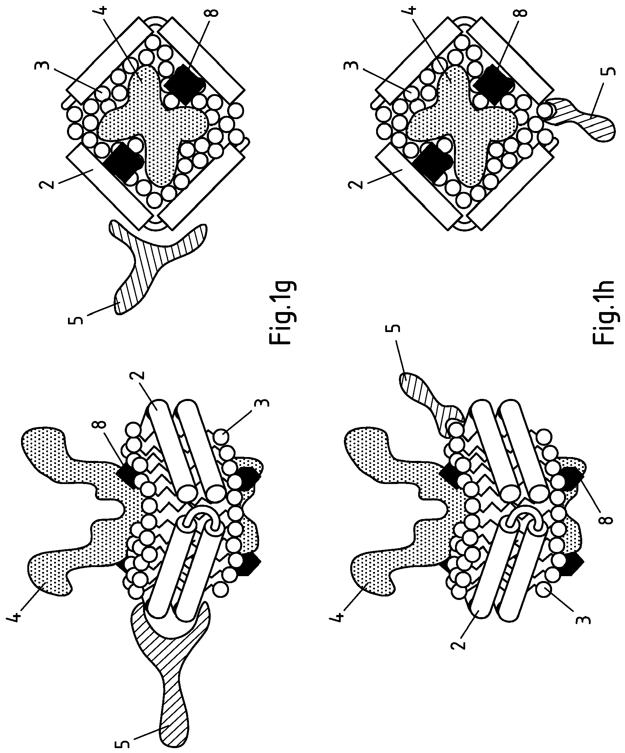

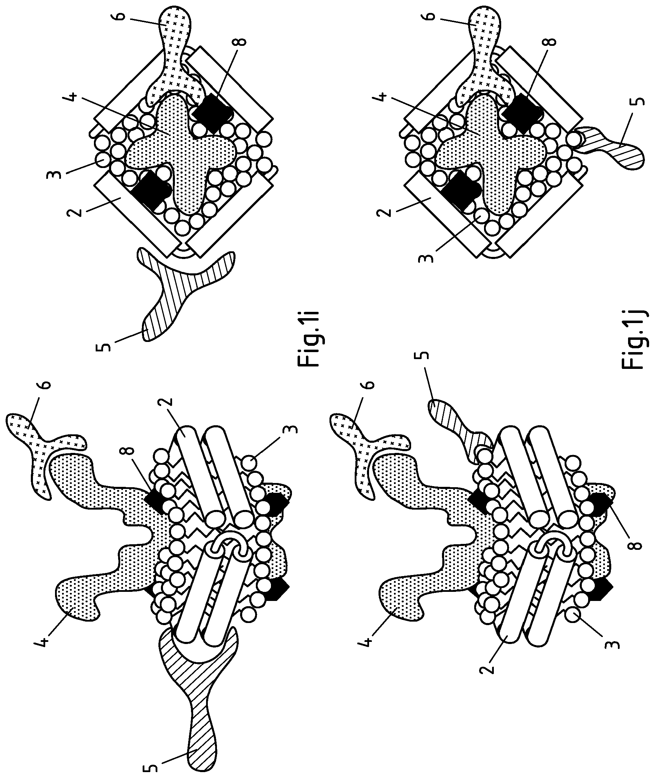

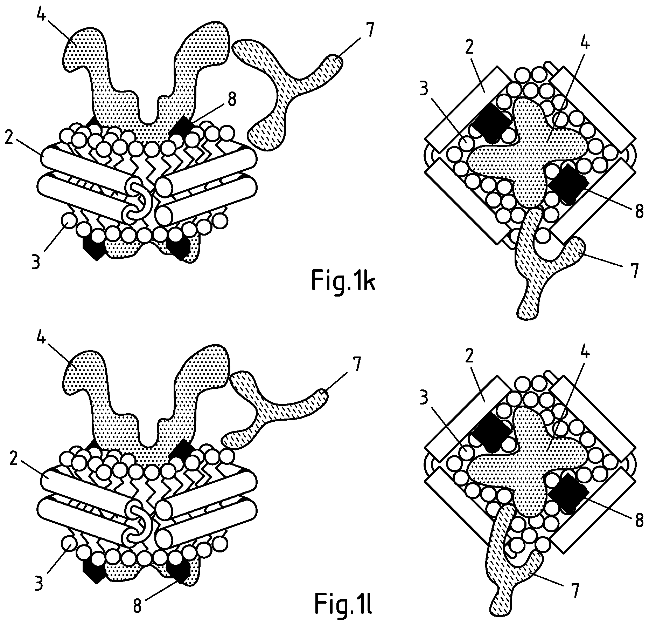

- Figures 1a to 11: are schematic illustrations of Salipro particles according to the invention in side view (left) and in top view (right). In Fig. 1a-1f a Salipro particle comprising a saposin-like protein (2), lipids (3), a membrane protein (4), a first binding agent (5) and optionally a second binding agent (6) or a combined (bispecific) first and second binding agent (7) is shown. In Fig. 1g-1l a Salipro particle comprising a saposin-like protein (2), lipids (3), a membrane protein (4), a drug molecule (8), a first binding agent (5) and optionally a second binding agent (6) or a combined (bispecific) first and second binding agent (7) is shown.
- Figure 2a: shows the results of the SEC analysis described in Example 2. These results demonstrate the formation of Biotin-Salipro-T2 particles.
- Figure 2b: shows the results of the streptavidin pull-down assay described in Example 2. In this assay, Biotin-Salipro-T2 particles were incubated with Streptavidin-Sepharose^{®} 4B affinity beads. Salipro-T2 particles without biotinylation were used as a control. This assay confirmed the successful production of Biotin-Salipro-T2 particles.

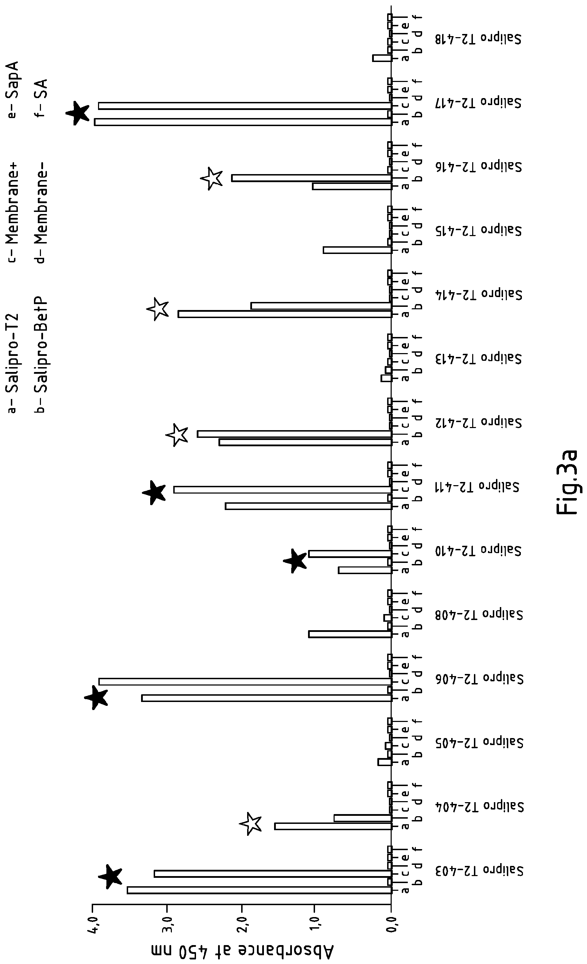

- Figure 3a: shows the results of the ELISA assay described in Example III.1, by which the binding specificity of the 14 selected Fab clones (14) was analyzed using Salipro-T2 particles (11) as coating antigen. As controls Salipro-BetP particles (13) containing the control hydrophobic agent BetP, T2-expressing membranes (membrane +) (12), control membranes not expressing T2 (membrane -), saposin A monomers (SapA), and streptavidin (SA) were used as coating antigens. The black stars indicate the Fab clones (14) which bound to Salipro-T2 particles and T2 expressing membranes. The white stars indicate the Fab clones (14) which bound to Salipro-T2 particles (11) and Salipro-BetP particles (13), but not to T2-expressing membranes and monomeric saposin A.
- Figure 3b: is a schematic illustration of the binding specificity of Fab clones 403, 406, 410, 411 and 417 as determined by the ELISA assay of Example III.1. The Fab clones (14) 403, 406, 410, 411 and 417 bound to Salipro-T2 particles (11) and T2 expressing membranes (12) when used as an ELISA coating (10). To detect the bound Fab clones a horseradish peroxidase coupled α-human kappa antibody (15) was used.
- Figure 3c: is a schematic illustration of the binding specificity of Fab clones 404, 412, 414 and 416 as determined by the ELISA assay of Example III.1. The Fab clones (14) 404, 412, 414 and 416 bound to Salipro-T2 particles (11) and Salipro-BetP particles (13) when used as an ELISA coating (10). To detect the bound Fab clones a horseradish peroxidase coupled α-human kappa antibody (15) was used.
- Figure 4a: is a schematic illustration of the SPR setup which was used in Example III.2. In this setup, an α-human kappa antibody (16) was immobilized on a CM5 chip to capture the purified Fab clones (14). This allowed to determine whether the Fab clones (14) can bind Salipro-T2 particles (11).
- Figure 4b: is another schematic illustration of the SPR setup which was used in Example III.2. In this other setup, an α-human kappa antibody (16) immobilized on a CM5 chip and which had captured a specific purified Fab clone (14) was incubated with Salipro-BetP particles (13) as analyte.
- Figure 5a: depicts the results of the SEC analysis for Fab clone 414 using Salipro-T2 particles as described in Example III.3.
- Figure 5b: depicts the results of the non-reducing SDS-PAGE analysis as described in Example III.3 for which the SEC peak fractions as shown in Figure 5a were used.
- Figure 6a: depicts the results of the SEC analysis for Fab clone 414 using Salipro-PANXl particles as described in Example III.3.
- Figure 6b: depicts the results of the reducing SDS-PAGE analysis as described in Example III.3 for which the SEC peak fractions as shown in Figure 6a were used.
- Figure 7a: depicts the results of the FSEC analysis for Fab clone 414 as described in Example III.3 which assessed whether Fab clone 414 binds to monomeric SapA and/or Salipro particles containing no hydrophobic agent.
- Figure 7b: depicts the results of the FSEC analysis for Fab clone 414 as described in Example III.3 which assessed whether Fab clone 414 binds to monomeric SapA.
- Figure 8a,b: reproduces Fig. 4A and 4B of Bruhn (2005), Biochem J 389 (15): 249-257. The sequences are provided as SEQ ID Nos 7-46 as indicated in table 1 above.

### EXAMPLES

In the following, the invention is further explained by examples. The invention, however, is as defined in the claims and generally described herein. It should not be limited to the embodiments shown for illustrative purposes in the examples below.

### I Biotinylation of Saposin A

To avoid chemical modifications of the hydrophobic agent in the Salipro particles which could potentially cause the destabilization of the hydrophobic agent and/or change its biological properties, the particle scaffold protein Saposin A was chosen as target for biotinylation using the NHS-ester activated reagent EZ-Link^{™} NHS-LC-LC-Biotin of ThermoFisher) with the aim of achieving about one biotinylation per Saposin A molecule.

The biotinylation of Saposin A was performed according to the manufacturer's instructions.

### II Production of Salipro T2 particles containing biotinylated Saposin A

The ion channel protein T2, a multispan transmembrane protein belonging to the class of mechanosensitive small conductance channels (MscS) and known to sense changes in external osmolarity, was chosen as hydrophobic agent to form Salipro T2 particles containing biotinylated Saposin A.

Typically, several copies of the Saposin A scaffold protein self-assemble around the Salipro particles. The number of Saposin A molecules for Salipro T2 particle is unknown. A cryoEM structure of a homotetrameric mitochondrial calcium uniporter (MCU) in Salipro particles is already solved and revealed the presence of six saposin scaffold proteins in the nanoparticle complex (Nguyen, Nam X., et al. "Cryo-EM structure of a fungal mitochondrial calcium uniporter." Nature 559.7715 (2018): 570-574). We therefore reasoned that one Salipro T2 particle theoretically contains at least six Saposin A scaffold proteins.

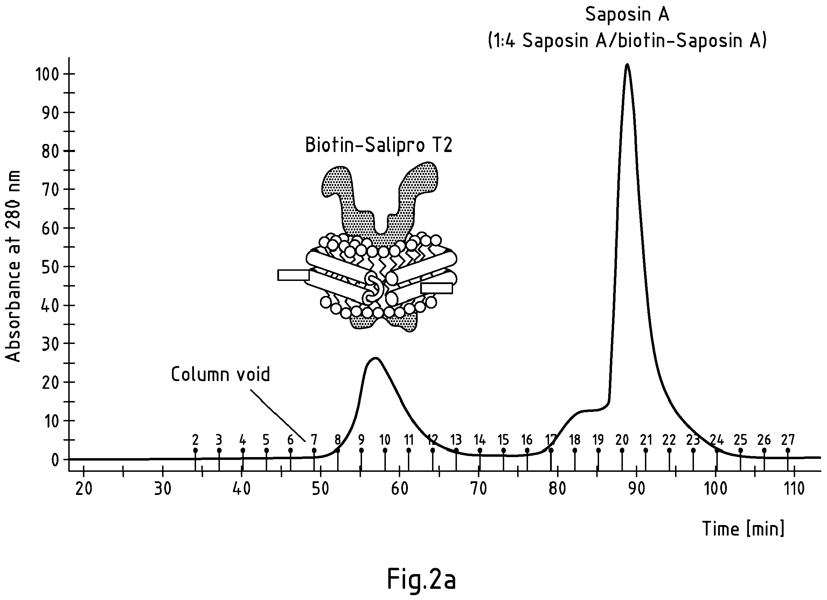

To produce Salipro T2 particles containing at least one biotin per nanoparticle, Saposin A that should contain at least one biotin per molecule was mixed with a four times excess of "plain" Saposin A (1:4 Saposin A/biotin-Saposin A). The 1:4 Saposin A/biotin-Saposin A mixture was then used to assemble biotin-Salipro-T2 particles by Size Exclusion Chromatography (SEC) using detergent-free HN-buffer (50 mM HEPES pH 7.5,150 mM NaCl, see Figure 2A).

Biotin-Salipro-T2 particles were collected, pooled, and concentrated in SEC fractions 9-11 (see Figure 2A) before being used in downstream assays.

To confirm the presence of biotin molecules on the Salipro nanoparticles, a streptavidin pull-down assay was performed. To this aim, Biotin-Salipro-T2 particles were incubated with a Streptavidin-Sepharose^{®} 4B affinity beads followed by extensive washing. The Biotin-Salipro-T2 particles were eluted from the affinity beads by adding SDS-PAGE sample buffer to the sample and by incubating the sample for 5 min at 95 °C. Salipro T2 particles without biotinylation were included as a control.

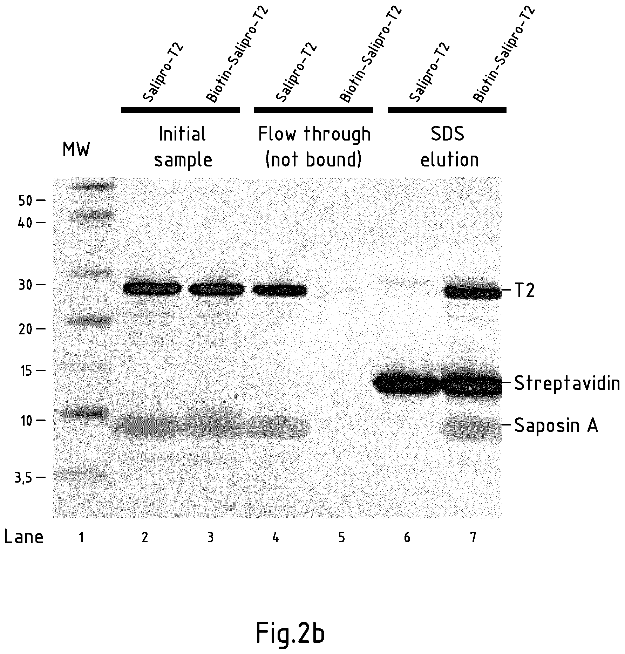

The resulting SDS-PAGE analysis showed equal amounts of starting material in the two samples (Figure 2B, lanes 2 and 3). The affinity bead wash fractions contained T2 and saposin scaffold protein in the control sample. This was not the case for the sample containing the Biotin-Salipro-T2 particles (Figure 2B, lane 4 and 5). Only Salipro T2 particles with biotinylated Saposin A were found to interact with the affinity beads (Figure 2B, lane 6 and 7). Streptavidin was also eluted from the affinity beads due to the harsh SDS elution conditions (Figure 2B, lane 6 and 7).

In conclusion, the saposin scaffold protein can be functionalized by using a standard protocol for NHS-ester biotinylation. This makes it possible to assemble biotin-labeled Salipro nanoparticles. Such particles can be used for phage display selection and also efficiently enable downstream analyses of T2 without the need of a tag or label attached to the native T2 protein.

### III Generation and characterization of first and second binders

The Salipro T2 biotinylated particles were used as an antigen in a phage display selection workflow.

The selection workflow included four rounds of selections using biotin-Salipro-T2 particles immobilized on magnetic streptavidin beads (Dynabeads M-280 streptavidin, Invitrogen). In selection round 1, streptavidin beads were first pre-incubated with 250 nM Saposin A to remove unwanted saposin A binders. Next, incubation with 150 nM biotin-Salipro-T2 was performed for 2.5 h in PBS at 4°C, followed by 5x 1ml washes using PBS supplemented with 0.01% digitonin at room temperature. In selection round 2, streptavidin beads were first pre-incubated with 100 nM Saposin A, then incubated with 50 nM biotin-Salipro-T2 for 1 h in PBS at 4°C, followed by 7x 1ml washes using PBS supplemented with 0.01% digitonin at room temperature. In selection round 3, streptavidin beads were first pre-incubated with 20 nM Saposin A, then incubated with 10 nM biotin-Salipro-T2 for 1 h in PBS at 4°C, followed by 9x 1ml washes using PBS supplemented with 0.01% digitonin at room temperature. In selection round 4, streptavidin beads were first pre-incubated with 10 nM Saposin A, then incubated with 2 nM biotin-Salipro-T2 for 1 h in PBS at 4°C, followed by 12x 1ml washes using PBS supplemented with 0.01% digitonin at room temperature.

All washing steps were performed in a 96-well plate using a KingFisher Flex robot (Thermo Fisher Scientific). Bound phages were recovered using digestion with trypsin (Gibco, Life Technologies), which cleaves a specific site located between the phage protein III and the displayed Fab. Subsequently, inactivation of trypsin was achieved with aprotinin (AppliChem).

Recovered phages were amplified overnight by infection of XL1-Blue *E*. *coli,* either on agar plates at 37 °C (round 1) or in solution at 30 °C (rounds 2-4). To produce an amplified phage stock, M13K07 (New England Biolabs) was used as helper phage. Precipitation of amplified phages was performed using PEG/NaCl, and the pellets were resuspended in PBS and used in the next round of selection.

The Fab clones against Salipro T2 particles which were identified in the phage display were subsequently screened in an ELISA assay. In this assay, Salipro-T2 particles (3 µg/ml) in PBS were coated onto well surfaces of a 96-well plate overnight at 4°C. As control coating antigens saposin A (1 µg/ml), Streptavidin (1 µg/ml) and a non-specific control antigen (1 µg/ml) were used. Filtered *E*. *coli* supernatants from Fab expressing clones were diluted 20x in PBS and incubated for 1h at 4°C, followed by 4x 1 ml washing in PBS. Fab binding was then detected using horseradish peroxidase-labelled α-human KAPPA antibody (Southern Biotech #9230) diluted 1:4000 in PBS and incubated for 1h at 4°C. TMB-ELISA substrate solution (Thermo Fisher Scientific) was used as a chromogenic substrate, and the reaction was stopped by adding 1 M H₂SO₄. The absorbance was measured at 450 nm.

Via this ELISA assay, 72 clones were identified, which bound specifically to Salipro-T2 particles. Sequencing of these clones revealed 18 unique clones. Cleared culture media from these clones, containing expressed Fab, were then used for an initial antigen binding test by SPR. Based on this SPR analysis, 14 Fab clones with the best relative affinity and selectivity for Salipro T2 particles were selected for protein purification.

### 1. Determination of the binding specificity

The binding specificity of the 14 purified Fabs was determined by an ELISA assay. To this aim, Salipro-T2 particles (3 µg/ml) in PBS were coated onto well surfaces of a 96-well plate overnight at 4°C. As control coating antigens, saposin A monomers (SapA, 1 µg/ml), Streptavidin (SA, 1 µg/ml), Salipro-BetP (3 µg/ml), crude membranes (15mg/ml) made from *E*. *coli* expressing T2 (membrane +) or not expressing T2 (membranes -) were used. Affinity purified Fab clones (5 µg/ml) in PBS were added and incubated for 1h at 4°C, followed by 4x 1 ml washing using PBS.

Fab binding was then detected using horseradish peroxidase-labelled α-human KAPPA antibody (Southern Biotech #9230) diluted 1:4000 in PBS and incubated for 1h at 4°C. TMB-ELISA substrate solution (Thermo Fisher Scientific) was used as a chromogenic substrate, and the reaction was stopped by adding 1 M H₂SO₄. The absorbance was measured at 450 nm.

Eleven Fab clones were confirmed to bind to Salipro-T2 particles (Figure 3A). Five of these (403, 406, 410, 411, 417) additionally bound to T2 expressed in membranes, confirming that these Fab binding epitopes are exposed on the native membrane anchored T2 channel (Figure 3B). In addition, four of the Salipro-T2-specific Fab clones (404, 412, 414, and 416) bound to Salipro-BetP control particles (Figure 3C) but did not bind to T2-expressing membranes or to monomeric saposin A.

### 2. Determination of the binding kinetics

Binding kinetic measurements of 14 purified Fab clones were performed using an SPR, Biacore T200 (GE Healthcare). An α-human KAPPA antibody (GE Healthcare # 28958325) was immobilized to a series S CM5 chip (GE Healthcare) using amine coupling according to the manufacturer's instructions. The running buffer used was HNG buffer supplemented with 0.5% BSA and all samples were diluted in this buffer before injection.

Purified Fab clones were captured to the chip by the immobilized α-human KAPPA antibody, whereby binding saturation was reached with a 30 µl/min flow rate. The flow rate was kept constant for all SPR experiments. This setup enabled binding measurements of Salipro T2 nanoparticles as injected analyte (schematically illustrated in figure 4A). Single-cycle kinetics experiments were performed. Five different concentrations of Salipro-T2 particles (0.2 - 50 nM) were injected, and a single injection (50nM) in the case of Salipro-BetP. Glycine-HCl, pH 2.5 was used for chip regeneration. Reaction rate kinetics constants were calculated using the Biacore T200 evaluation software 3.1 using the 1:1 Langmuir binding model. Response curve sensorgrams were obtained after removing the response from the reference channel and a reference cycle (running buffer instead of Fab). Transient spikes, for example from air bubbles, were also removed.

K_{D} values for Salipro T2-specific interactions were in the 0.3-36 nM range for all the 14 isolated Fab clones. Fab clones that bound to native membrane-bound T2 channels in ELISA assays (Fab clones 404, 406, 410, 411, 417) all bound to Salipro-T2 with high affinity K_{D} values in the 0.3-3 nM range (Table 2). A single Salipro-BetP antigen injection was used as a control to evaluate Fab clone binding to the particle scaffold (Figure 4B) and clearly showed binding of the Salipro-BetP particles to Fab clones 404, 412, 414, 416 (Tabelle 2).

**Table 2: K_{D} values of the 14 purified Fabs. Avidity contributions to affinity constants may have occurred due to multivalent antigens. "K_{D} app" values should only be considered as apparent affinity since only a single antigen injection was performed. "--" stands for "no binding detected".**

| | **Salipro- T2** | | | **Salipro-BetP** |
|---|---|---|---|---|
| **Clone name** | **ka (1/Ms)** | **kd (1/s)** | **K_{D} (M)** | **K_{D} app (M)** |
| Salipro- T2-403 | 7,1E+04 | 2,3E-05 | 3,2E-10 | -- |
| Salipro- T2-404 | 2,5E+05 | 4,6E-04 | 1,9E-09 | 3,4E-09 |
| Salipro- T2-405 | 7,4E+04 | 2,7E-03 | 3,6E-08 | -- |
| Salipro- T2-406 | 1,3E+05 | 2,7E-05 | 2,0E-10 | -- |
| Salipro- T2-408 | 2,7E+05 | 6,2E-04 | 2,3E-09 | -- |
| Salipro-T2-410 | 3,5E+05 | 1,0E-03 | 2,9E-09 | -- |
| Salipro-T2-411 | 7,7E+04 | 2,3E-04 | 3,0E-09 | -- |
| Salipro-T2-412 | 4,2E+05 | 5,4E-04 | 1,3E-09 | 1,4E-09 |
| Salipro-T2-413 | 1,2E+05 | 1,1E-03 | 9,7E-09 | -- |
| Salipro-T2-414 | 5,3E+05 | 2,2E-04 | 4,2E-10 | 1,3E-09 |
| Salipro-T2-415 | 6,8E+04 | 4,0E-04 | 6,0E-09 | -- |
| Salipro-T2-416 | 2,9E+05 | 3,6E-04 | 1,2E-09 | 2,3E-09 |
| Salipro-T2-417 | 2,3E+05 | 6,1E-05 | 2,6E-10 | -- |
| Salipro-T2-418 | 4,5E+05 | 1,9E-03 | 4,2E-09 | -- |

### 3. Further characterization of the binding specificity of Fab clone 414

To further characterize the binding specificity of Fab clone 414, a SEC peak shift assay was used to investigate Fab 414 interactions. If the interaction is stable, a SEC peak shift is expected, both due to increased particle size (shifts the SEC peak to the left in the graph) and due to increased particle mass (increases the SEC peak signal height) when compared to a control sample not incubated with the Fab.

To this aim, Salipro-T2 particles were first incubated with and without an excess of Fab clone 414 and then subjected to SEC analysis (Superdex 200 Increase 5/150 GL: 50 mM HEPES (pH 7.5), 150 mM NaCl, 5% glycerol).

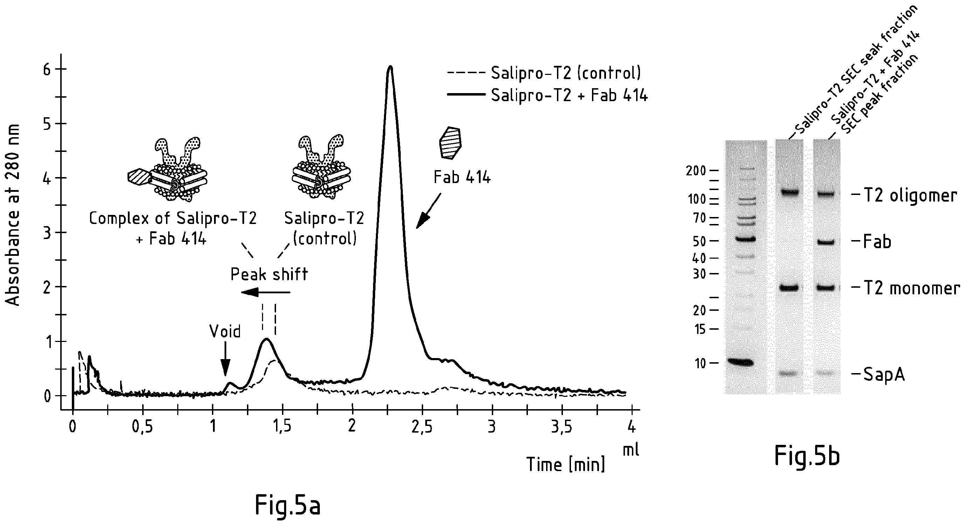

In this SEC analysis, Fab clone 414 displayed a stable interaction to Salipro T2 particles, shifting the SEC peak in protein size and amount (Figure 5A). In addition, non-reducing SDS-PAGE analyses of SEC peak fractions from both the Salipro-T2/Fab complex sample and the Salipro-T2 control sample confirmed the presence of Fab clone 414 only in the SEC peak shift (Figure 5B).

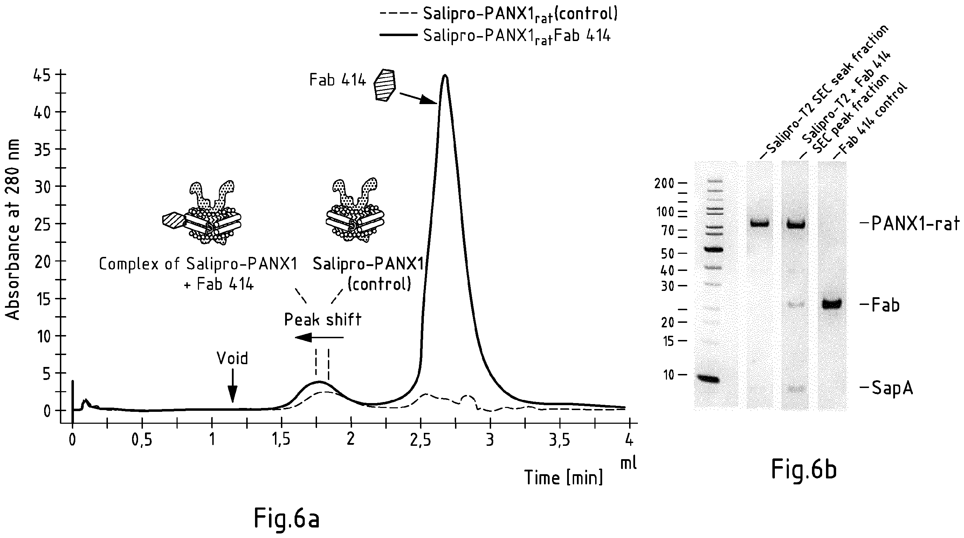

The same SEC experiment was performed with Salipro particles containing another membrane protein, pannexin 1 (PANX1), using a Superose 6 Increase 5/150 GL (50 mM HEPES (pH 7.5), 150 mM NaCl, 5% glycerol). PANX1 was reconstituted into Salipro particles by the crude membrane method (for details see WO 2018/033 647 A1 and EP 3 725 304 A1). Purified Salipro-PANXl particles were incubated with or without Fab clone 414 followed by SEC analysis. The data clearly showed a stable interaction between Fab clone 414 and Salipro-PANXl particles as illustrated by the SEC peak shift (Figure 6A) and the presence of the Fab clone 414 in the peak-shifted SEC fraction as revealed by reducing SDS-PAGE (Figure 6B).

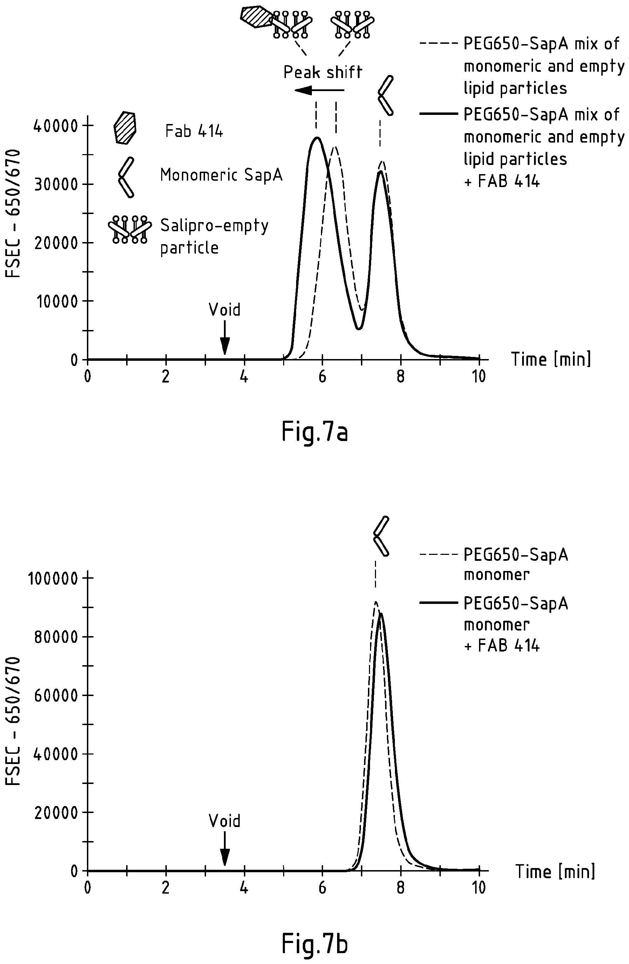

To further understand the binding specificity of Fab clone 414, we explored its binding to monomeric saposin A and lipid-only Salipro nanoparticles using the SEC peak shift assay described above. Since an excess of Fab will cover the saposin SEC peaks when measured using protein absorption at 280 nm, a new setup was developed. To this aim, Saposin A was fluorescently labelled (PEG650-SapA) and then used to make a sample containing both saposin A monomers and Salipro lipid only nanoparticles. The mixed population of fluorescently labelled saposin A was then incubated with an excess of the Fab clone 414 and analysed by SEC using a fluorescent detector (FSEC, excitation 650 nm/Emission 670 nm). An apparent FSEC peak shift was observed for the lipid-only Salipro nanoparticles, but not for monomeric saposin A (figure 7A). As a control, fluorescent monomeric saposin A was then also used as the only component incubated with an excess of Fab clone 414, leading to no FSEC peak shift (Figure 7B

## Claims

1. A method comprising determining a full or partial 2D or 3D structure of
- a hydrophobic agent, and/or
- a second agent interacting with the hydrophobic agent, and/or
- a binding site between
- a biologically active agent and a hydrophobic agent, or
- a biologically active agent and lipids, or
- a biologically active agent a hydrophobic agent and lipids;
the method comprising the following steps:
a) providing a particle comprising the following components
- a saposin-like protein or a derivative form thereof;
- lipids;
- a hydrophobic agent wherein the hydrophobic agent is different from the lipids; and
- a first binding agent that is bound to
- the saposin-like protein or the derivative form thereof; and/or
- the lipids;
- optionally a second binding agent that is bound to the hydrophobic agent and optionally lipids; and
- optionally a biologically active agent interacting with the hydrophobic agent and/or the lipids;
b) determining the full or partial 2D or 3D structure of the particle of step a) using a structural biology method for structure elucidation.

2. The method of claim 1, wherein the structural biology method of step b) is selected from the group consisting of nuclear magnetic resonance spectroscopy (NMR), X-ray crystallography, small-angle X-ray scattering (SAXS), surface-plasmon resonance (SPR), and electron microscopy (EM), in particular negative-stain electron microscopy and/or cryo-electron microscopy (cryo-EM).

3. The method of claim 1 or 2, wherein
(a) the particle of step a) is at least 20 kDa, preferably at least 40 kDa, more preferably 60 kDa and most preferably at least 80 kDa in size; and/or
(b) the structure of the particle is determined in step b) at a resolution higher than 25 Å, preferably higher than 15 Å, more preferably higher than 10 Å, even preferably higher than 5 Å and most preferably higher than 3 Å.

4. The method according to any of claims 1 to 3, wherein the particle and/or its components recited in step a) are defined as in any one of claims 7 to 14.

5. The method according to any of claims 1 to 4, wherein the method comprises as additional step c) the use of the full or partial 2D or 3D structure determined in step b) for analyzing binding properties, determining structure-activity relationships, structure-based drug design, in particular for influencing or designing a biologically active agent, in particular a drug molecule, binding to the hydrophobic agent or to the hydrophobic agent and lipids.

6. A method for studying or identifying a biologically active agent that binds to a hydrophobic agent, the method comprising:
a. obtaining one or more partial or full 2D or 3D structures, preferably one or more partial or full 3D structures of a particle comprising
- a saposin-like protein or a derivative form thereof;
- lipids;
- a hydrophobic agent wherein the hydrophobic agent is different from the lipids;
- a first binding agent wherein the binding agent is bound to
- the saposin-like protein or the derivative form thereof; and/or
- the lipids;
- optionally a second binding agent wherein the second binding agent is bound to the hydrophobic agent and optionally the lipids; and
- optionally a drug molecule interacting with the hydrophobic agent and/or the lipids;
b. modeling the binding of at least one biologically active agent to the hydrophobic agent using the one or more 2D or 3D structures obtained in step a.;
c. selecting one or more biologically active agents which are modeled to selectively bind to the hydrophobic agent in step b.; and
d. optionally, confirming the selective binding of the one or more biologically active agent selected in step c. to the hydrophobic agent in the particle of step a. by contacting the particle with the selected one or more biologically active agents and measuring the binding between the hydrophobic agent in the particle and the selected one or more drug candidates and/or the biological activity of the hydrophobic agent.

7. A particle comprising
- a saposin-like protein or a derivative form thereof;
- lipids;
- a hydrophobic agent wherein the hydrophobic agent is different from the lipids;
- a first binding agent wherein the first binding agent is bound to
- the saposin-like protein or the derivative form thereof and/or
- the lipids;
- optionally
- a second binding agent wherein the second binding agent is bound to the hydrophobic agent; and/or
- a drug molecule interacting with the hydrophobic agent and/or the lipids.

8. The particle according to claim 7, wherein the first binding agent is selected from the group consisting of an antibodies or antigen-binding portions thereof, polymers, affibodies, affilins, anticalins, atrimers, avimers, peptides, bicyclic peptides, Cys-knots, designed Ankyrin repeat proteins (DARPins), FN3 scaffolds, Fynomers, Kunitz domains, OBodies, monobodies, anticalins, affibodies, aptamers, megabodies, nanobodies and wherein preferably the first binding agent is an antibody or an antigen-binding portion thereof such as a polyclonal antibody, a monoclonal antibody, a multispecific antibody, a chimeric antibody, a single-chain antibody, a Fab fragment, a Fab' fragment, a F(ab')₂ fragment, an Fd fragment, an Fv, a single-chain Fv (scFv), a disulfide-linked Fv (sdFv), or a fragment comprising a VL or VH domain.

9. The particle according to claim 7 or 8, wherein the first binding agent is capable of binding to particle-contained saposin-like protein or a particle-contained derivative form thereof and substantially not to the corresponding non-particle-contained saposin-like protein or the non-particle-contained derivative form thereof.

10. The particle according to any one of claims 7 to 9, wherein the first binding agent contains a further functional moiety apart from an antigen binding moiety, in particular a capture moiety, a multimerization moiety, a labeling moiety and combinations thereof.

11. The particle according to any one of claims 7 to 10, wherein the lipids are selected from the group consisting of viral lipids, archaeal lipids, eukaryotic lipids, prokaryotic lipids, and mixtures thereof.

12. The particle according to any one of claims 7 to 11, wherein the saposin-like protein is saposin A, saposin B, saposin C, saposin D or a derivative form thereof, which is capable of forming particles according to any one of claims 7 to 11.

13. The particle according to any one of claims 7 to 12, wherein the derivative form of the saposin-like protein is
i. a protein having at least 20 % sequence identity to the full length sequence of a sequence selected from SEQ ID NO. 1-51; in particular wherein said protein is amphipathic, forms at least one alpha helix, and is capable of self-assembling together with lipids into a particle according to claim 1; and/or
ii. a protein comprising a sequence selected from SEQ ID NO. 1-51
- in which 1 to 40 amino acids have been deleted and/or substituted; and/or
- in which additional amino acids have been inserted or added.

14. The particle according to any one of claims 7 to 13, wherein the hydrophobic agent is a hydrophobic biomolecule, preferably a hydrophobic biomolecule selected from the group consisting of a membrane protein, an integral transmembrane protein, an integral monotopic membrane protein, a peripheral membrane protein, an amphitropic protein in a lipid-bound state, a lipid-anchored protein, a chimeric protein with a fused hydrophobic and/or transmembrane domain.

15. Use of a first binding agent capable of binding to saposin-like protein or a derivative form thereof and/or to lipids in a particle according to any one of claims 7 to 14 for
(a) increasing the particle size, influencing the orientation of the particle on a solid support, the symmetry and/or the rigidity of the particle to determine the 2D or 3D structure, preferably the 3D structure, of a hydrophobic agent contained in the particle.
(b) multimerizing the particles, labeling the particles and/or for capturing an additional cargo molecule.

16. Use of a particle according to any one of claims 7 to 14 in a pharmaceutical composition, in particular a vaccine formulation, a therapeutic or prophylactic method, in particular in a method to prevent, treat or lessen the severity of a disease, a diagnostic method, or a cosmetic treatment.
